# EUROPEAN PATENT SPECIFICATION

(11) **EP 3 160 480 B1**
(45) Date of publication and mention of the grant of the patent: **09.09.2020**
(21) Application number: 15742101.7
(22) Date of filing: 30.06.2015
(51) Int. Cl.: A61K 35/28, A61P 19/02

(54) **MESENCHYMAL STROMAL CELLS FOR TREATING RHEUMATOID ARTHRITIS**
MESENCHYMALE STROMAZELLEN ZUR BEHANDLUNG VON RHEUMATOIDER ARTHRITIS
CELLULES STROMALES MÉSENCHYMATEUSES POUR LE TRAITEMENT DE L'ARTHRITE RHUMATOIDE

(30) Priority: 30.06.2014 EP 14175096
(43) Date of publication of application: 03.05.2017
(73) Proprietor: TiGenix, S.A.U., 28760 Tres Cantos (Madrid) (ES)
(72) Inventor: DALEMANS, Wilfried, E-28760 Tres Cantos (Madrid) (ES); LOMBARDO, Eleuterio, E-28760 Tres Cantos (Madrid) (ES); DEKKER, Robert, E-28760 Tres Cantos (Madrid) (ES)
(74) Representative: Lasar, Andrea Gisela
(86) International application number: PCT/IB2015/054922
(87) International publication number: WO 2016/001845

(56) References cited:
- WO-A1-2012/123401
- WO-A1-2014/207679
- WO-A2-2007/039150
- US-A1- 2012 201 791
- Anonymous: "TiGenix reports positive Phase IIa study results in refractory rheumatoid arthritis with allogeneic stem cell product Cx611", , 22 April 2013 (2013-04-22), XP055143269, Retrieved from the Internet: URL:http://www.tigenix.com/public/uploads/ pdf/en/f5177d809eca202.40307502_TiGenix Cx611 Phase IIa results Final.pdf [retrieved on 2014-09-29]
- Anonymous: "Cx611 in RA", Press Releases, 22 April 2013 (2013-04-22), pages 1-48, XP055144050, WWW Retrieved from the Internet: URL:http://www.tigenix.com/en/download/?s= rNXU8lvo7ZUjEybwArESBX4S%2BkEKDlBvQYuoUTu% 2FQzzXtBQzMAl7TEC3l8DkwthFj%2BYyaSGvZW9cSU 6K10OQDQ%3D%3D [retrieved on 2014-10-02]
- JORGE PAZ RODRIGUEZ ET AL: "Autologous stromal vascular fraction therapy for rheumatoid arthritis: rationale and clinical safety", INTERNATIONAL ARCHIVES OF MEDICINE, BIOMED CENTRAL LTD, LONDON UK, vol. 5, no. 1, 8 February 2012 (2012-02-08), page 5, XP021119262, ISSN: 1755-7682, DOI: 10.1186/1755-7682-5-5
- HONG KYUNG LEE ET AL: "Preclinical Efficacy and Mechanisms of Mesenchymal Stem Cells in Animal Models of Autoimmune Diseases", IMMUNE NETWORK, vol. 14, no. 2, 1 January 2014 (2014-01-01), page 81, XP055214029, ISSN: 1598-2629, DOI: 10.4110/in.2014.14.2.81
- JIN-HEE KIM ET AL: "Paradoxical effects of human adipose tissue-derived mesenchymal stem cells on progression of experimental arthritis in SKG mice", CELLULAR IMMUNOLOGY, vol. 292, no. 1-2, 6 November 2014 (2014-11-06), pages 94-101, XP055214027, ISSN: 0008-8749, DOI: 10.1016/j.cellimm.2014.10.005
- PHILIPPE BOURIN ET AL: "Stromal cells from the adipose tissue-derived stromal vascular fraction and culture expanded adipose tissue-derived stromal/stem cells: a joint statement of the International Federation for Adipose Therapeutics and Science (IFATS) and the International Society for Cellular Therapy (ISCT)", CYTOTHERAPY, vol. 15, no. 6, 1 June 2013 (2013-06-01), pages 641-648, XP055143289, ISSN: 1465-3249, DOI: 10.1016/j.jcyt.2013.02.006
- ICHIM T E ET AL: "Autologous stromal vascular fraction cells: A tool for facilitating tolerance in rheumatic disease", CELLULAR IMMUNOLOGY, ACADEMIC PRESS, SAN DIEGO, CA, US, vol. 264, no. 1, 1 January 2010 (2010-01-01), pages 7-17, XP027114463, ISSN: 0008-8749 [retrieved on 2010-04-08]
- SCHAEFFLER ANDREAS ET AL: "Concise review: Adipose tissue-derived stromal cells - Basic and clinical implications for novel cell-based therapies", STEM CELLS, ALPHAMED PRESS, DAYTON, OH, US, vol. 25, no. 4, 1 April 2007 (2007-04-01), pages 818-827, XP009133278, ISSN: 1066-5099, DOI: 10.1634/STEMCELLS.2006-0589 [retrieved on 2007-04-01]
- Anonymous: "-TiGenix Press Release 2013", EN -NL) 26 July 2013 -TiGenix Completes EUR 6.5 million Capital Increase, 1 November 2013 (2013-11-01), XP055143981, Retrieved from the Internet: URL:http://www.tigenix.com/en/pages/11/201 3 [retrieved on 2014-10-02]
- OLGA DELAROSA ET AL: "Mesenchymal stem cells as therapeutic agents of inflammatory and autoimmune diseases", CURRENT OPINION IN BIOTECHNOLOGY, vol. 23, no. 6, 1 December 2012 (2012-12-01), pages 978-983, XP055096416, ISSN: 0958-1669, DOI: 10.1016/j.copbio.2012.05.005
- GONZALEZ-REY E ET AL: "Human adipose-derived mesenchymal stem cells reduce inflammatory and T cell responses and induce regulatory T cells in vitro in rheumatoid arthritis", ANNALS OF THE RHEUMATIC DISEASES, BRITISH MEDICAL ASSOCIATION, LONDON, GB, [Online] vol. 69, no. 1, 1 January 2010 (2010-01-01), pages 241-248, XP008152922, ISSN: 0003-4967, DOI: 10.1136/ARD.2008.101881 Retrieved from the Internet: URL:http://ard.bmjjournals.com/> [retrieved on 2009-01-05]

## Description

### BACKGROUND

Rheumatoid arthritis (RA) is an autoimmune disease caused by loss of immunologic self tolerance that leads to chronic inflammation of the joints and subsequent cartilage destruction and bone erosion. The crucial process underlying disease initiation is the induction of autoimmunity to collagen-rich joint components followed by the subsequent events associated with the evolving immune and inflammatory responses. Treating rheumatoid arthritis is a crucial challenge for the health systems of the developed world and increasingly for developing countries. The incidence of rheumatoid arthritis is between 0.5% to 1% of the general population worldwide with little variation of prevalence among countries. It affects over 21 million people worldwide (UN World Population Database, 2004), and has a great impact on the patient's quality of life and gives rise to significant economic and social costs. The economic burden associated to the treatment of RA is formidable for any health care economy and it has been estimated that the combined annual economic cost of this disease is up to €45.5 billion across Europe.

Traditionally, RA has been treated with non-steroidal anti-inflammatory drugs, glucocorticoids and non-biologics-DMARDs. The current pharmacological management of rheumatoid arthritis generally involves early intervention with synthetic disease modifying anti-rheumatic drugs (DMARDs) either singly or in combination. If inflammation cannot be adequately suppressed by these means, typically biologic DMARDs targeting the proinflammatory cytokine TNF are employed. During the last decade, the introduction of biologies has improved therapeutic options for rheumatoid arthritis patients. Biologies, initially directed to neutralize TNF-α, have experienced a big growth over the last number of years regarding both the number of new molecules and the target to which they are directed. Lack of efficacy in some patients, non-tolerability or recurrent secondary infections are factors that have contributed to the need for the development of new therapies. Nonetheless, rheumatoid arthritis remains an unmet clinical need where approximately 20-40% of rheumatoid arthritis patients do not have an adequate response to anti-TNF.

Mesenchymal stromal cell therapy has been proposed as an approach for the treatment of inflammatory and autoimmune diseases such as rheumatoid arthritis. Mesenchymal stromal cells (MSCs) are non-hematopoietic stromal cells that are able to differentiate into mesenchymal tissues such as bone, cartilage, muscle, ligament, tendon, and adipose. MSCs can be easily isolated from tissues such as bone marrow or adipose tissue and rapidly expanded in culture. MSCs can modulate immune responses, showing antiproliferative and anti-inflammatory capacities which is the basis for their use as potential candidate therapies for the treatment of immune-mediated inflammatory conditions. However there exists a need for establishing effective clinical dosages and dosage regimens for cell therapies in the field of rheumatoid arthritis.

The article "TiGenix reports positive Phase IIa study results in refractory rheumatoid arthritis with allogeneic stem cell product Cx611", available under URL: http://www.tigenix.com/public/uploads/pdf/en/f5177d809eca202.40307502_TiGenix Cx611 Phase IIa results Final.pdf discusses that allogeneic mesenchymal stem cells may be used to treat refractory rheumatoid arthritis.

The press release "Cx611 in RA", available under URL: http://www.tigenix.com/en/download/?s=rNXU8lvo7ZUjEybwArESBX4S%2BkEKD1BvQYuoUTu %2FQzzXtBQzMA17TEC318DkwthFj%2BYyaSGvZW9cSU6K100QDQ%3D%3D relates to the treatment of a severe grade of rheumatoid arthritis in patients which were refractory to biologics and DMARDs.

### SUMMARY OF THE INVENTION

It has been found that administration of mesenchymal stromal cells (MSCs), in particular human adipose tissue derived stromal cells (hASCs), may be useful in the treatment of rheumatoid arthritis.

The invention therefore provides a composition comprising mesenchymal stromal cells (MSCs), for use in treating rheumatoid arthritis in a human subject, wherein the disease is moderate rheumatoid arthritis having a CDAI score of >10 to ≤22 and wherein the subject is first treated at no more than about 12 months from first diagnosis or onset of disease symptoms..

The disclosure also provides the use of mesenchymal stromal cells (MSCs) in the manufacture of a medicament for treating rheumatoid arthritis in a subject.

The disclosure also provides a method of treating rheumatoid arthritis in a subject in need thereof, comprising administering a composition comprising mesenchymal stromal cells to the subject.

In one aspect, the invention provides a composition comprising mesenchymal stromal cells (MSCs), for use in treating rheumatoid arthritis in a human subject with moderate rheumatoid arthritis having a CDAI score of >10 to ≤22, and optionally having a DAS28 score of >3.2 to ≤5.1 and/or a RAPID3 score of >6 to ≤12, wherein the subject is first treated at no more than about 12 months from first diagnosis or onset of disease symptoms.

### BRIEF DESCRIPTION OF DRAWINGS

Figure 1: Arthritis score (A) and incidence (B) at days 21-54 after collagen injection. "CIA" = no treatment; "CIA+ASC d15,d18,d21" = intravenous administration of ASCs at days 15, 18 and 21 after collagen injection.
Figure 2: Arthritis score at days 1-59 (day 1 = first ASC dose). ASCs were administered intravenously at days 1, 3 and 5 only, or continued thereafter at days 8, 15, 22 and 29.
Figure 3: Effect of three intravenous administrations of ASCs (8.000, 40.000, 200.000 or 1 million ASCs per dose) at days 1, 3 and 5 (day 1 = first ASC dose).
Figure 4: Effect of three intravenous administrations of ASCs (8.000, 40.000, 200.000 or 1 million ASCs per dose) at days 1, 8 and 15 (day 1 = first ASC dose).
Figure 5: Levels of (A) IL6; (B) IL17; (C) IL23; and (D) CD3+CD25bright (regulatory T cells) at day 22 in healthy mice, untreated mice with arthritis ("CIA"), and mice with arthritis treated with ASCs by intravenous administration ("CIA+ASC IV").
Figure 6: Bone mineral density (A) and trabecular mineral density (B) at day 50 in untreated mice with arthritis ("CIA"), and mice with arthritis treated with ASCs by intravenous administration ("CIA+ASC").
Figure 7: Bone mineral density (A) and trabecular mineral density (B) at day 50 in untreated mice with arthritis (left bar), and mice with arthritis treated with ASCs by intralymphatic administration (right bar).
Figure 8: Effect of intralymphatic administration in early arthritis. Arthritis score (A and B) and paw volume (C and D).
Figure 9: Levels of regulatory T cells (CD4+CD25+FoxP3+cells) in the spleen (A) and lymph node (B) of healthy mice, untreated mice with arthritis ("CIA"), and mice with arthritis after intralymphatic administration of ASCs ("CIA+ASC IL").
Figure 10: Levels of CD4 cells expressing IL 10 (TR1 cells) in the spleen (A) and lymph node (B) of healthy mice, untreated mice with arthritis ("CIA"), and mice with arthritis after intralymphatic administration of ASCs ("CIA+ASC IL").
Figure 11: DAS28 response in ASC-treated human patient subpopulation, recorded over several visits (V4-V9), starting with DAS28 of <4.5 (lower line) or >5.5 (upper line) at baseline (A) and normalised to baseline (B).
Figure 12: CDAI levels in ASC-treated human patient subpopulation, recorded over several visits (V4-V9), starting with CDAI of <22 (lower line) or >22 and <40 (upper line) at baseline (A) and normalised to baseline (B).
Figure 13: ASC-treated patients achieving ACR20 (A), ACR50 (B), and ACR70 (C) throughout the study (ITT population).
Figure 14: ASC-treated patients with good EULAR response throughout the study (A); with low disease activity (DAS28-ESR<3.2) (B); in clinical remission (DAS28-ESR<2.6) (C); and showing DAS28-ESR change from baseline (D) throughout the study (ITT population).

### DEFINITIONS

As used herein, the following terms and phrases shall have the meanings set forth below. Unless defined otherwise, all technical and scientific terms used herein have the same meaning as commonly understood to one of ordinary skill in the art to which this invention belongs.

The articles "a" and "an" refer to one or to more than one (i.e., to at least one) of the grammatical object of the article. By way of example, "an element" means one element or more than one element.

The term "about" when used in relation to a value relates to the value ±10%.

By "adipose tissue" is meant any fat tissue. The adipose tissue may be brown or white adipose tissue, derived from, for example, subcutaneous, omental/visceral, mammary, gonadal, or other adipose tissue site. Preferably, the adipose tissue is subcutaneous white adipose tissue. The adipose tissue may comprise a primary cell culture or an immortalized cell line. The adipose tissue may be from any organism having fat tissue. In some embodiments, the adipose tissue is mammalian, and in further embodiments the adipose tissue is human. A convenient source of adipose tissue is liposuction surgery. However, it will be understood that neither the source of adipose tissue nor the method of isolation of adipose tissue is critical to the invention. If cells as described herein are desired for autologous transplantation into a subject, the adipose tissue will be isolated from that subject.

"Adipose tissue-derived stromal cells (ASCs)" refers to MSCs that originate from adipose tissue, generally from human adipose tissue (hASCs).

"Refractory" shall be taken to mean having no significant clinical benefit when used in the treatment of a diseases e.g. no significant improvement or amelioration of symptoms, subsequent relapse of disease or resulting in toxicity.

The term "biological medicinal product" shall be taken to mean a protein or nucleic acid-based pharmaceutical substance for therapeutic use, which is typically produced by means other than direct extraction from a native (nonengineered) biological source.

The term "cells/kg" as used herein shall be taken to mean the number of cells (e.g. MSC) administered per kilogram of patient body weight.

The term "constitutively" is understood to mean the expression of a gene without any specific induction.

The term "culture" refers to the growth of cells, organisms, multicellular entities, or tissue in a medium. The term "culturing" refers to any method of achieving such growth, and may comprise multiple steps. The term "further culturing" refers to culturing a cell, organism, multicellular entity, or tissue to a certain stage of growth, then using another culturing method to bring said cell, organism, multicellular entity, or tissue to another stage of growth. A "cell culture" refers to a growth of cells in vitro. In such a culture, the cells proliferate, but they do not organize into tissue per se. A "tissue culture" refers to the maintenance or growth of tissue, e.g., explants of organ primordial or of an adult organ in vitro so as to preserve its architecture and function. A "monolayer culture" refers to a culture in which cells multiply in a suitable medium while mainly attached to each other and to a substrate. Furthermore, a "suspension culture" refers to a culture in which cells multiply while suspended in a suitable medium. Likewise, a "continuous flow culture" refers to the cultivation of cells or explants in a continuous flow of fresh medium to maintain cell growth, e.g. viability. The term "conditioned media" refers to the supernatant, e.g. free of the cultured cells/tissue, resulting after a period of time in contact with the cultured cells such that the media has been altered to include certain paracrine and/or autocrine factors produced by the cells and secreted into the culture. A "confluent culture" is a cell culture in which all the cells are in contact and thus the entire surface of the culture vessel is covered, and implies that the cells have also reached their maximum density, though confluence does not necessarily mean that division will cease or that the population will not increase in size.

The term "culture medium" or "medium" is recognized in the art, and refers generally to any substance or preparation used for the cultivation of living cells. The term "medium", as used in reference to a cell culture, includes the components of the environment surrounding the cells. Media may be solid, liquid, gaseous or a mixture of phases and materials. Media include liquid growth media as well as liquid media that do not sustain cell growth. Media also include gelatinous media such as agar, agarose, gelatin and collagen matrices. Exemplary gaseous media include the gaseous phase that cells growing on a petri dish or other solid or semisolid support are exposed to. The term "medium" also refers to material that is intended for use in a cell culture, even if it has not yet been contacted with cells. In other words, a nutrient rich liquid prepared for bacterial culture is a medium. Similarly, a powder mixture that when mixed with water or other liquid becomes suitable for cell culture may be termed a "powdered medium". "Defined medium" refers to media that are made of chemically defined (usually purified) components. "Defined media" do not contain poorly characterized biological extracts such as yeast extract and beef broth. "Rich medium" includes media that are designed to support growth of most or all viable forms of a particular species. Rich media often include complex biological extracts. A "medium suitable for growth of a high density culture" is any medium that allows a cell culture to reach an OD600 of 3 or greater when other conditions (such as temperature and oxygen transfer rate) permit such growth. The term "basal medium" refers to a medium which promotes the growth of many types of microorganisms which do not require any special nutrient supplements. Most basal media generally comprise four basic chemical groups: amino acids, carbohydrates, inorganic salts, and vitamins. A basal medium generally serves as the basis for a more complex medium, to which supplements such as serum, buffers, growth factors, lipids, and the like are added. Examples of basal media include, but are not limited to, Eagles Basal Medium, Minimum Essential Medium, Dulbecco's Modified Eagle's Medium, Medium 199, Nutrient Mixtures Ham's F-10 and Ham's F-12, McCoy's 5A, Dulbecco's MEM/F-I 2, RPMI 1640, and Iscove's Modified Dulbecco's Medium (IMDM).

The terms "comprise" and "comprising" are used in the inclusive, open sense, meaning that additional elements may be included.

The term "expanded" as used herein when referring to cells shall be taken to have its usual meaning in the art, namely cells that have been proliferated in vitro. A MSC can be expanded to provide a population of cells that retain at least one biological function of the MSC, typically the ability to adhere to a plastic surface, under standard culture conditions. The expanded population of cells may retain the ability to differentiate into one or more cell types.

The term "including" is used herein to mean "including but not limited to". "Including" and "including but not limited to" are used interchangeably.

"Marker" refers to a biological molecule whose presence, concentration, activity, or phosphorylation state may be detected and used to identify the phenotype of a cell.

"Mesenchymal stromal cells (also referred to herein as "MSCs") are multipotent stromal cells, *i.e.* they are cells which are capable of giving rise to multiple different types of cells.

A "patient", "subject" or "host" to be treated by the subject method may mean either a human or non-human animal.

The term "pharmaceutical composition" refers to a composition intended for use in therapy. The compositions for use in the invention are pharmaceutical compositions, intended for use in treating rheumatoid arthritis. The compositions for use in the invention may include, in addition to MSCs, non-cellular components. Examples of such non-cellular components include, but are not limited to, cell culture media, which may comprise one or more of proteins, amino acids, nucleic acids, nucleotides, co-enzyme, anti-oxidants and metals.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient.

The term "phenotype" refers to the observable characteristics of a cell, such as size, morphology, protein expression, etc.

"Proliferation" refers to an increase in cell number. "Proliferating" and "proliferation" refer to cells undergoing mitosis.

As used herein, the term "solution" includes a pharmaceutically acceptable carrier or diluent in which the MSCs used in the invention remain viable.

The term "substantially pure", with respect to MSC populations, refers to a population of cells in which at least about 75%, at least about 85%, at least about 90%, or at least about 95%, by number of the cells are MSCs. In other words, the term "substantially pure", with respect to MSC populations, refers to a population of cells that contains less than about 20%, less than about 10%, or less than about 5%, by number of lineage committed cells.

"Support" as used herein refers to any device or material that may serve as a foundation or matrix for the growth of adipose tissue-derived stromal cells.

"Therapeutic agent" or "therapeutic" refers to an agent capable of having a desired biological effect on a host. Chemotherapeutic and genotoxic agents are examples of therapeutic agents that are generally known to be chemical in origin, as opposed to biological, or cause a therapeutic effect by a particular mechanism of action, respectively. Examples of therapeutic agents of biological origin include growth factors, hormones, and cytokines. A variety of therapeutic agents are known in the art and may be identified by their effects. Certain therapeutic agents are capable of regulating cell proliferation and differentiation. Examples include chemotherapeutic nucleotides, drugs, hormones, non-specific (non-antibody) proteins, oligonucleotides (e.g., antisense oligonucleotides that bind to a target nucleic acid sequence (e.g., mRNA sequence)), peptides, and peptidomimetics.

"First diagnosis" as used herein shall be taken to mean the first diagnosis by a qualified medical practitioner (e.g. rheumatologist, general practitioner, MD, nurse practitioner) of rheumatoid arthritis, preferably according to clinically accepted criteria.

"Disease duration" shall be taken to mean time since onset of disease symptoms.

### INDICATIONS

The present invention provides novel uses of mesenchymal stromal cells (hereinafter also referred to as "MSCs") in the treatment of rheumatoid arthritis, as well as compositions of mesenchymal stromal cells suitable for such uses. The rheumatoid arthritis is moderate rheumatoid arthritis.

The diagnosis and severity classification are preferably carried out using clinically accepted criteria such as those established by American College of Rheumatology (ACR) or The European League Against Rheumatism (EULAR) e.g. Disease Activity Score 28-joint count (DAS28), Clinical Disease Activity Index (CDAI), and the Routine Assessment of Patient Index Data 3 (RAPID3). In one embodiment a DAS28 score >3.2 to ≤5.1 is considered moderate and a DAS28 score >5.1 is considered as severe. In an alternative embodiment a CDAI score >10 to ≤22 is considered moderate and a CDAI score >22 is considered as severe. In a further alternative embodiment a RAPID3 score >6 to ≤12 is considered moderate and a RAPID3 score >12 is considered as severe.

Thus, the invention provides MSCs for use in the treatment of moderate rheumatoid arthritis in a patient, wherein the disease has a DAS28 score of >3.2 to ≤5.1, a CDAI score of >10 to ≤22 and/or a RAPID3 score of >6 to ≤12.

Such patients typically have 'early rheumatoid arthritis'. Historically, early rheumatoid arthritis was considered as less than 5 years disease, but by the early-1990s this had decreased to 24 months or less, with greater emphasis on the first 12 months. Currently, many rheumatologists wish to see patients with early RA at the first available opportunity. The proportion of rheumatologists who wish to see patients within 6 weeks from symptom onset doubled from 9% in the year 1997 to 17% in 2003, though not all patients were seen so rapidly (D. L. Scott, Early rheumatoid arthritis, British Medical Bulletin 2007; 81 and 82: 97-114).

Thus, the invention provides MSCs for use in the treatment of moderate rheumatoid arthritis in a patient, wherein the disease has a CDAI score of >10 to ≤22, optionally a DAS28 score of >3.2 to ≤5.1 and/or a RAPID3 score of >6 to ≤12, and wherein the rheumatoid arthritis is early rheumatoid arthritis, *e.g*. of not more than about 6 months from first diagnosis or onset of disease symptoms, or not more than about 12 months from first diagnosis or onset of disease symptoms. The examples show that particularly good therapeutic effects are achieved in this subpopulation.

The disclosure also provides MSCs for use in the treatment of severe rheumatoid arthritis in a patient, wherein the disease has a DAS28 score of >5.1, a CDAI score of >22 and/or a RAPID3 score of >12.

The MSCs are used in treating rheumatoid arthritis in patients not more than about 6 months from first diagnosis or not more than about 12 months from first diagnosis.

In a preferred embodiment of the invention the MSCs are used in treating rheumatoid arthritis in patients having not more than about 6 months disease duration, not more than about 12 months disease duration, not more than about 18 months disease duration, not more than about 2 years disease duration, not more than about 3 years disease duration, not more than about 4 years disease duration, not more than about 5 years disease duration.

Typically the MSCs are used in treating rheumatoid arthritis in patients during an acute phase of the disease.

The term "acute phase" in the context of rheumatoid arthritis shall be taken to mean a patient experiencing significant inflammation of one or more joints, as opposed to only mild or moderate inflammation. The acute phase is also referred to as "flares" in the art.

Methods for diagnosing acute rheumatoid arthritis are known in the art and may include the analysis of Erythrocyte sedimentation rate (ESR) and/or serum C-reactive protein (CRP) levels wherein e.g. ESR of 50 or above is considered to be acute.

MSCs have been found to confer an immune-modulatory effect, particularly at sites of inflammation. During acute flares of rheumatoid arthritis (*i.e.* during the acute phase), which are characterised by raised inflammatory markers, exogenously delivered MSCs are therapeutic through modulating the inflammatory immune responses and dampening the consequent inflammatory cytokine environment. Thus, MSCs are particularly effective in treating rheumatoid arthritis during the acute phase, before irreversible processes have established. Thus, the invention provides MSCs for use in treating rheumatoid arthritis in patients during an acute phase of the disease.

In a preferred embodiment of the invention the MSCs are used in treating rheumatoid arthritis in patients refractory to at least one, two or three therapies for treatment of rheumatoid arthritis. Typically the patient has received said treatment for at least about 6, 12, 18, 24 or 36 weeks and still presents symptoms of active disease. In other words, even after treatment for at least about 6, 12, 18, 24 or 36 weeks, the severity of the disease is not ameliorated.

Said therapies may include one or more of disease-modifying antirheumatic drugs (DMARDs), non-steroidal anti-inflammatories (NSAIDs), biological medicinal products and corticosteroids. NSAIDs including but are not limited to Cox-2 inhibitors, diclofenac, ibuprofen, naproxen, celecoxib, mefenamic acid, etoricoxib, indometacin and aspirin. Corticosteroids include but are not limited to triamcinolone, cortisone, prednisone, and methylprednisolone. Disease-modifying antirheumatic drugs (DMARDs), include but are not limited to auranofin, choroquine, cyclosporine, cyclophosphamide, gold preparations, hydroxychloroquine sulphate, leflunomide, methotrexate, penicillamine, sodium aurothiomalate, sulfasalazine.

In one embodiment the patient is treatment refractory to at least 2 or 3 DMARDs, preferably selected from the group consisting of hydroxychloroquine, leflunomide, methotrexate, minocycline, and sulfasalazine (e.g., methotrexate and hydroxychloroquine; methotrexate and leflunomide; methotrexate and sulfasalazine; sulfasalazine and hydroxychloroquine; methotrexate, hydroxychloroquine and sulfasalazine).

These drugs are targeted to specific anti-inflammatory molecular pathways. In contrast, MSCs provide a more physiological approach, which involves modulating multiple inflammatory pathways and providing a global dampening of pathological hyper-inflammatory responses. The MSCs also provide a mechanism for negative feedback loops which would enable a physiological balance of pro- and anti-inflammatory cytokines to be struck, whereas this is not possible with a mere delivery of predetermined doses of anti-inflammatory drugs. Thus, MSCs are particularly effective in treating rheumatoid arthritis in patients who are treatment refractory to any of the anti-inflammatory drugs. In one embodiment it is particularly preferred that the patient is treatment refractory to at least methotrexate. In one embodiment the patient is treatment refractory to at least 2 or 3 DMARDs, comprising methotrexate and one or more DMARDs selected from the group consisting of hydroxychloroquine, leflunomide, minocycline, and sulfasalazine.

In a further embodiment it is preferred that the patient is treatment refractory to a combination therapy comprising methotrexate and at least one, two or all of corticosteroids, non-steroidal anti-inflammatories, and DMARDs (e.g. leflunomide and/or sulfasalazine), e.g. methotrexate and prednisone; or methotrexate, sulfasalazine and prednisone. In one embodiment it is particularly preferred that the patient is treatment refractory to at least leflunomide. In a further embodiment it is preferred that the patient is treatment refractory to at least both of leflunomide and methotrexate.

In a further embodiment, it is preferred that the patient is treatment refractory to methotrexate and cyclosporine. These patients have a high likelihood to be non-responders to TNF therapy.

In a further embodiment it is preferred that the patient is treatment refractory to a combination therapy comprising methotrexate, leflunomide and at least one, two or all of corticosteroids, non-steroidal anti-inflammatories, and sulfasalazine, e.g. methotrexate, leflunomide, a DMARD (e.g. hydroxychloroquine and/or sulfasalazine), a corticosteroid (e.g. prednisone).

Typically the patient has received therapy with at least methotrexate for at least about 6, 12, 18, 24 or 36 weeks and has active disease. In other words, even after treatment for at least about 6, 12, 18, 24 or 36 weeks, the severity of the disease is not ameliorated.

Optionally the patients may have received biological medicinal products. Biological medicinal products, include but are not limited to selective costimulation modulators, TNF-α inhibitors, IL-1 inhibitors, IL-6 inhibitors & B-cell targeted therapies. Typically, the patient is refractory to at least one biological treatment, for example a TNF-α inhibitor. Accordingly, in one aspect of the invention the patient is refractory to at least one biological treatment which is a TNF-α inhibitor, typically Adalimumab (Humira), Certolizumab (Cimzia), Etanercept (Enbrel), Golimumab (Simponi), or Infliximab (Remicade). Typically, the patient is refractory to at least two biological treatments, optionally at least one of which may be a TNF-α inhibitor. In one embodiment the patient is treatment refractory to at least methotrexate and a TNF-α inhibitor; wherein it is particularly preferred that said TNF-α inhibitor is selected from the group comprising Adalimumab, Etanercept & Infliximab.

In a further embodiment the patients are treatment refractory to at least leflunomide and a TNF-α inhibitor; wherein it is particularly preferred that said TNF-α inhibitor is selected from the group comprising Adalimumab, Etanercept & Infliximab. In a further embodiment the patient is treatment refractory to at least leflunomide, methotrexate and a TNF-α inhibitor; wherein it is particularly preferred that said TNF-α inhibitor is selected from the group comprising Adalimumab, Etanercept & Infliximab.

The invention also provides MSCs for use in the treatment of rheumatoid arthritis in patients during an acute phase of the disease, wherein the patient is treatment refractory to methotrexate. Preferably the patient is treatment refractory to a further DMARD, for example, cyclosporine.

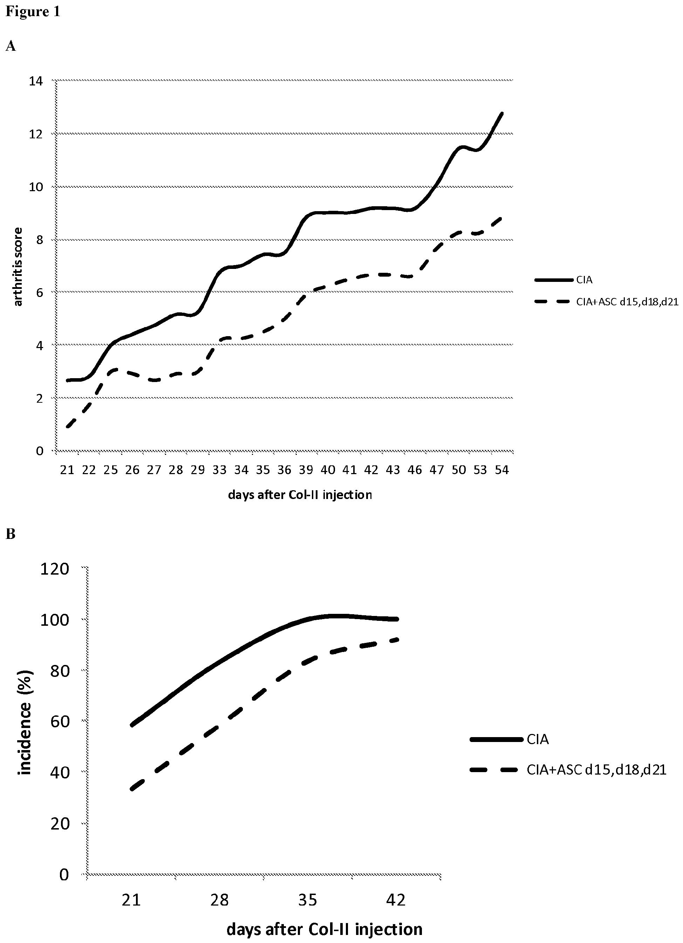

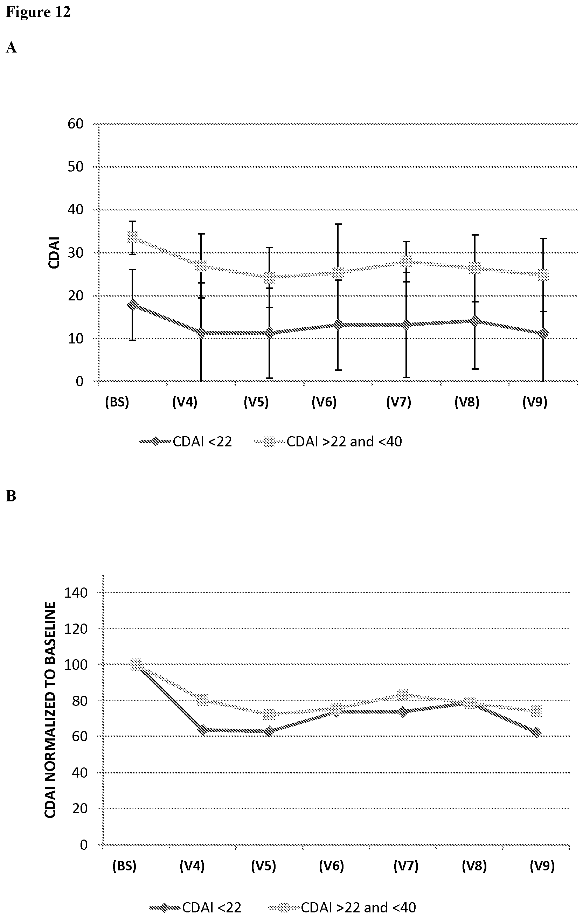

The examples show that arthritis can be treated by administering ASCs, and that the therapeutic effect is particularly good when ASCs are first administered at early/moderate stages of the disease. Surprisingly, beneficial long-term therapeutic effects are observed even after treatment with ASCs is discontinued. Benefits were observed over the entire period of ASC administration, for example 3 or 7 administrations with an interval of one day or one week (Figures 1-4). Repeated administration of ASCs, for example 3 administrations with an interval of one day or one week, were shown to be beneficial in human patients even after 6 months from ASC administration, especially in those with moderate rheumatoid arthritis (Figure 12).

Accordingly, in one aspect the invention provides MSCs for use in the treatment of rheumatoid arthritis in a subject wherein the MSCs are administered repeatedly, *i.e.* two or more doses are administered, for example at least 3 doses, at least 4, 5, 6, 7, 8, 9, or 10 doses. The interval between doses can be daily, weekly, or monthly. The MSCs can be administered over a period of no more than 1 week (*e.g*. with daily intervals, such as at days 1 and 3, or days 1,3 and 5, or days 1, 3, 5 and 7), or MSCs can be administered over a period of 1 week or longer (*e.g*. at days 1, 8 and 15, where day 1 denotes the first dose of MSCs), for example at least 4 weeks, at least 1 months, at least 6 weeks, at least 8 weeks, at least 2 months, at least 3 months, at least 4 months, at least 5 months, at least 6 months, at least 9 months, or at least 12 months. In each of these embodiments, the rheumatoid arthritis is moderate rheumatoid arthritis with a CDAI score of >10 to ≤22, optionally a DAS28 score of >3.2 to ≤5.1 and/or a RAPID3 score of >6 to ≤12, as measured at baseline (*i.e.* at the first ASC dose). Typically, the MSCs are ASCs, for example human eASCs, optionally administered intralymphatically, for example by intralymphatic injection.

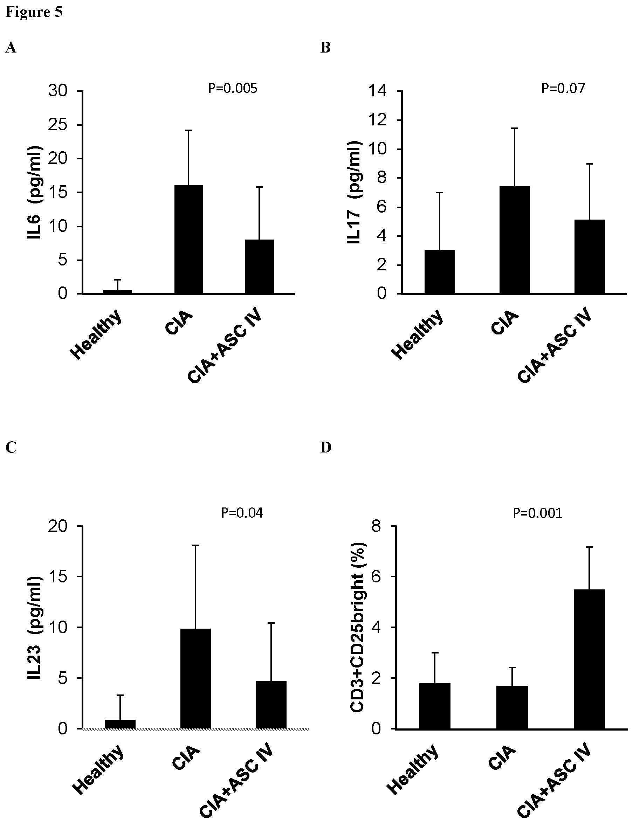

The examples also show that treatment with ASCs significantly reduces the levels of proinflammatory cytokines IL6, IL17, and IL23, and increases the levels of regulatory T cells (CD3+CD25bright), compared to untreated mice (Fig. 5). Accordingly, in one embodiment the invention provides MSCs for use in the treatment of rheumatoid arthritis in a subject wherein administration of MSCs reduces the levels of systemic (*e.g.* measured in plasma from peripheral blood; relevant methods are well known to the person skilled in the art, *e.g*. ELISA) IL6, IL17 and/or IL23 to statistically significant levels, compared to untreated subjects. In some embodiments, the systemic levels of IL6, IL17 and/or IL23 levels are 80% or less, *e.g.* 75% or less, 70% or less, 65% or less, 60% or less, 55% or less or 50% or less compared to typical systemic levels of the respective cytokines in untreated subjects. Alternatively or additionally, in some embodiments the levels of systemic (*e.g*. measured in the cell fraction of peripheral blood; relevant methods are well known to the person skilled in the art, *e.g*. FACS) CD3+CD25bright cells is increased to statistically significant levels, compared to untreated subjects. In some embodiments, the level of systemic CD3+CD25bright cells is 2-fold or more, *e.g*. 2.5-fold or more, compared to typical systemic levels of CD3+CD25bright cells in untreated subjects.

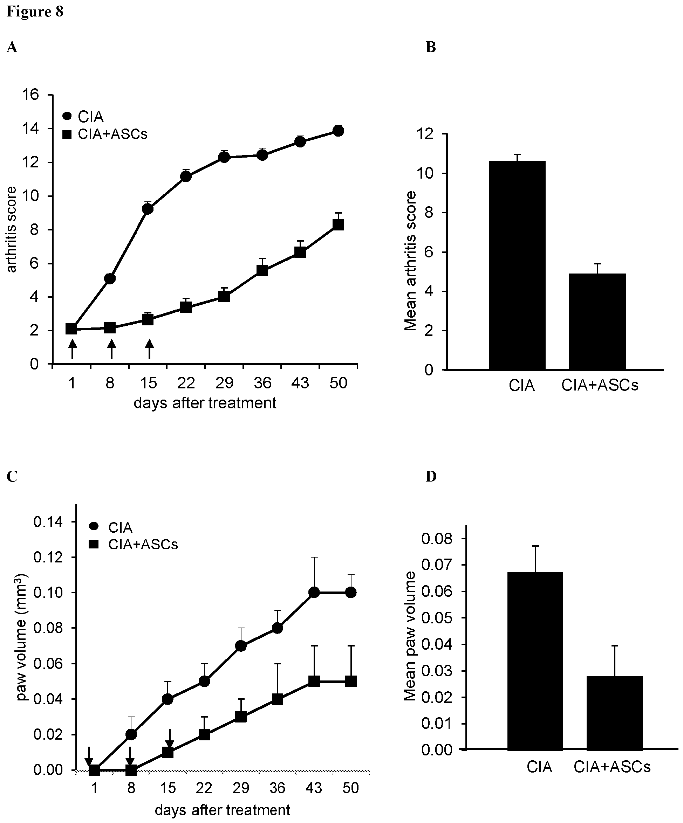

Intralymphatic administration has been shown to be beneficial (Figure 8). Accordingly, in any of the embodiments described above, the MSCs, typically ASCs, can be administered by the intralymphatic route, for example by intralymphatic injection.

### CELLS FOR USE IN THE INVENTION

MSCs are undifferentiated stromal cells having the capacity to differentiate to other cells, and are typically derived from connective tissue, and are thus non-hematopoietic cells. The term "connective tissue" refers to tissue derived from mesenchyme and includes several tissues which are characterized in that their cells are included within the extracellular matrix. Among the different types of connective tissues, adipose and cartilaginous tissues are included. In one embodiment, the MSCs are from the stromal fraction of the adipose tissue. In another embodiment, the MSCs are obtained from chondrocytes e.g. from hyaline cartilage. In another embodiment, the MSCs are obtained from skin. Also, in another embodiment, the MSCs are obtained from bone marrow. Alternative sources of MSCs include but are not limited to periosteum, dental pulp, spleen, pancreas, ligament, tendon, skeletal muscle, umbilical cord and placenta.

The MSCs can be obtained from any suitable source and from any suitable animal, including humans. Typically, said cells are obtained from post-natal mammalian connective tissues. In a preferred embodiment, the MSCs are obtained from a source of connective tissue, such as the stromal fraction of adipose tissue, hyaline cartilage, bone marrow, skin etc. Also, in a particular embodiment, the MSCs are from a mammal, e.g., a rodent, primate, etc., preferably, from a human. Typically, the MSCs are obtained from the stromal fraction of human adipose tissue, *i.e.* they are adipose tissue-derived stromal cells (ASCs).

The MSCs are adherent to plastic under standard culture conditions.

MSCs are undifferentiated multipotent cells, having the capacity to differentiate into or towards somatic cells such as mesodermal cells (e.g. adipose, chondrocytes, osteoblasts) and optionally into or towards endodermal and/or ectodermal cell types or lineages. Typically the cells have the capacity to differentiate into or towards at least two or all cell types selected from the group consisting of adipocytic, chondroblastic and osteoblastic lineages.

In one embodiment the MSCs may be stem cells, typically adipose tissue derived stem cells. Typically the MSCs (i) do not express markers specific from APCs; (ii) do not express IDO constitutively (iii) do not significantly express MHC II constitutively. Typically expression of IDO or MHC II may be induced by stimulation with IFN-γ.

Typically the MSCs may express one or more (*e.g.*two or more, three or more, four or more, five or more, six or more, seven or more, eight or more, nine or more, or ten or more (*e.g*. up to 13)) of the markers CD9, CD10, CD13, CD29, CD44, CD49A, CD51, CD54, CD55, CD58, CD59, CD90 and CD105. For example, the MSCs may express one or more (*e.g*. two, three or all) of the markers CD29, CD59, CD90 and CD105, e.g. CD59 and/or CD90.

Typically the MSCs may not express one or more (*e.g*. two or more, three or more, four or more, five or more, six or more, seven or more, eight or more, nine or more, or ten or more (*e.g*. up to 15)) of the markers Factor VIII, alpha-actin, desmin, S-100, keratin, CD11b, CD11c, CD14, CD45, HLAII, CD31, CD34, CD45, STRO-1 and CD133, e.g. the MSCs do not express one or more (*e.g*. two, three or all) of the markers CD34, CD45, CD31 and CD14, e.g. CD31 and/or CD34.

### EXPANDED MSC

In one embodiment the MSCs are *in vitro* culture expanded MSCs or the *in vitro* culture expanded progeny thereof (hereinafter both are referred to as expanded MSCs or "eMSCs"). Methods for the preparation of eMSCs are known in the art, for example as described in WO2007039150. eMSCs retain several phenotypic characteristics of MSC, e.g. the eMSCs are adherent to plastic under standard culture conditions and retain an undifferentiated phenotype.

eMSCs are undifferentiated multipotent cells, having the capacity to differentiate into somatic cells such as mesodermal cells. Whereas MSCs have the capacity to differentiate towards at least one or more specialized cell lineages such as but not limited to adipocytic, chondroblastic and osteoblastic lineages; typically in eMSCs this capacity to differentiate is reduced or may even be absent e.g. whereas a MSCs may differentiate towards at least the osteogenic and adipocytic lineages, the eMSCs derived therefrom may differentiate only towards the adipocytic lineage. This may be advantageous for therapeutic applications of the cells, where the cells may be administered to patients as it can reasonably be expected that unanticipated and potentially harmful differentiation of eMSCs will be less likely to occur.

In one embodiment the eMSCs may be the progeny of stem cells. Typically the eMSCs (i) do not express markers specific from APCs; (ii) do not express IDO constitutively (iii) do not significantly express MHC II constitutively. Typically expression of IDO or MHC II may be induced by stimulation with IFN-γ.

Typically the eMSCs may express one or more (*e.g*. two or more, three or more, four or more, five or more, six or more, seven or more, eight or more, nine or more, or ten or more (*e.g*. up to 13)) of the markers CD9, CD10, CD13, CD29, CD44, CD49A, CD51, CD54, CD55, CD58, CD59, CD90 and CD105, e.g. the MSCs may express one or more (*e.g*. two, three or all) of the markers CD29, CD59, CD90 and CD105, e.g. CD59 and/or CD90.

Typically the eMSCs may not express one or more (*e.g*. two or more, three or more, four or more, five or more, six or more, seven or more, eight or more, nine or more, or ten or more (*e.g*. up to 15)) of the markers Factor VIII, alpha-actin, desmin, S-100, keratin, CD11b, CD11c, CD14, CD45, HLAII, CD31, CD34, CD45, STRO-1 and CD133, e.g. the MSCs do not express one or more (*e.g*. two, three or all) of the markers CD34, CD45, CD31 and CD14, e.g. CD31 and/or CD34. Furthermore the MSCs may optionally not express the marker STRO-1.

### CELL POPULATIONS

In one aspect the present invention provides populations of MSCs and/or eMSCs for therapeutic uses as described herein, these populations may hereinafter be referred to as "cell populations for use in the invention". Typically, the MSCs are expanded human ASCs, typically allogeneic expanded human ASCs. Typically the cell populations for use in the invention are a homogenous or substantially homogenous population of MSCs and/or eMSCs. Cell populations for use in the invention comprise or comprise essentially of MSCs and/or eMSCs, however cell populations for use in the invention may also comprise other cell types. Accordingly in one embodiment the invention provides cell populations for use in the invention in which at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, of the cells are MSCs and/or eMSCs.

Typically the cell populations for use in the invention are a culture expanded population of MSCs, comprising or comprising essentially of eMSCs, however cell populations for use in the invention may also comprise other cell types. Accordingly in one embodiment the invention provides cell populations for use in invention in which at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, of the cells are eMSCs. In one embodiment, the eMSCs are eASCs, for example human eASCs. In a particular embodiment, the cells are allogeneic with respect to the subject to be treated.

Typically a cell population for use in the invention may have the capacity to differentiate towards at least one or more specialized cell lineages such as but not limited to adipocytic, chondroblastic and osteoblastic lineages. In one embodiment a cell population for use in the invention may have the capacity to differentiate into or towards at least two or all cell types selected from the group consisting of adipocytic, chondroblastic and osteoblastic lineages. However in an alternative embodiment this capacity to differentiate is reduced or may even be absent e.g. whereas a MSC may differentiate towards at least the osteogenic and adipocytic lineages, the eMSC population derived therefrom may differentiate only towards the adipocytic lineage. This may be advantageous for therapeutic applications of the cells, where the cells may be administered to patients as it can reasonably be expected that unanticipated and potentially harmful differentiation of eMSCs will be less likely to occur.

Typically a cell population for use in the invention may express one or more (*e.g*. two or more, three or more, four or more, five or more, six or more, seven or more, eight or more, nine or more, or ten or more(*e.g*. up to 13)) of the markers CD9, CD10, CD13, CD29, CD44, CD49A, CD51, CD54, CD55, CD58, CD59, CD90 and CD105, e.g. the MSCs may express one or more (*e.g*. two, three or all) of the markers CD29, CD59, CD90 and CD105, e.g. CD59 and/or CD90. Accordingly in one embodiment the invention provides a cell population for use in the invention in which at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, of the cells express one or more (e.g. two or more, three or more, four or more, five or more, six or more, seven or more, eight or more, nine or more, or ten or more (*e.g*. up to 13)) of the markers CD9, CD10, CD13, CD29, CD44, CD49A, CD51, CD54, CD55, CD58, CD59, CD90 and CD105, e.g. the MSCs may express one or more (*e.g*. two, three or all) of the markers CD29, CD59, CD90 and CD105, e.g. CD59 and/or CD90. In an alternative embodiment the invention provides a cell population for use in the invention in which the level of expression of one or more (*e.g*. two or more, three or more, four or more, five or more, six or more, seven or more, eight or more, nine or more, or ten or more (*e.g*. up to 13)) of the markers CD9, CD10, CD13, CD29, CD44, CD49A, CD51, CD54, CD55, CD58, CD59, CD90 and CD105 is at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, e.g. the cell population for use in the invention may express one or more (*e.g*. two, three or all) of the markers CD29, CD59, CD90 and CD105, e.g. CD59 and/or CD90 at the afore-mentioned level.

Typically a cell population for use in the invention may not express one or more (*e.g*. two or more, three or more, four or more, five or more, six or more, seven or more, eight or more, nine or more, or ten or more (*e.g*. up to 15)) of the markers Factor VIII, alpha-actin, desmin, S-100, keratin, CD11b, CD11c, CD14, CD45, HLAII, CD31, CD34, CD45, STRO-1 and CD133, e.g. the MSCs do not express one or more (*e.g*. two, three or all) of the markers CD34; CD45; CD31; CD14 e.g. CD31 and/or CD34. Furthermore the MSCs may optionally not express the marker STRO-1.

Accordingly in one embodiment the invention provides a cell population for use in the invention in which at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, of the cells do not express one or more (*e.g*. two or more, three or more, four or more, five or more, six or more, seven or more, eight or more, nine or more, or ten or more (*e.g*. up to 15)) of the markers Factor VIII, alpha-actin, desmin, S-100, keratin, CD11b, CD11c, CD14, CD45, HLAII, CD31, CD34, CD45, STRO-1 and CD133, e.g. the MSCs may express one or more (*e.g.* two, three or all) of the markers CD34, CD45, CD31 and CD14, e.g. CD31 and/or CD34. In an alternative embodiment the invention provides cell populations for use in the invention in which the level of expression of one or more (*e.g*. two or more, three or more, four or more, five or more, six or more, seven or more, eight or more, nine or more, or ten or more (*e.g*. up to 15)) of the markers Factor VIII, alpha-actin, desmin, S-100, keratin, CD11b, CD11c, CD14, CD45, HLAII, CD31, CD34, CD45, STRO-1 and CD133 is below at least about 35%, at least about 30%, at least about 25%, at least about 20%, at least about 25%, at least about 5%, at least about 1%, e.g. the cell populations for use in the invention may express one or more (*e.g*. two, three or all) of the markers CD34, CD45, CD31 and CD14, e.g. CD31 and/or CD34 at the afore-mentioned level.

In some embodiments MSCs or eMSCs are pre-stimulated in order to enhance one or more of their proliferation capacity, migration capacity, survival capacity, therapeutic effect and inmunoregulatory properties. Typically, at least about 40% (e.g. at least about 50%, at least about 60%, at least about 70%, at least about 80%, at least about 85%, at least about 90%, at least about 95% at least about 96%, at least about 97%, at least about 98%, or at least about 99%) of the cell populations for use in the invention are pre-stimulated in order to enhance one or more of their proliferation capacity, migration capacity, survival capacity, therapeutic effect and inmunoregulatory properties. In some embodiments, pre-stimulation may be achieved by contacting the MSCs with a cytokine. In some embodiments of the invention, pre-stimulation may be achieved by contacting the MSCs with IFN-γ.

### COMPOSITIONS

In one embodiment the present invention provides compositions comprising cell populations for use in methods of treatment according to the present invention. It is preferred that said composition is a pharmaceutical composition. A composition for use in the invention may include a substantially pure population of MSCs or eMSCs, for example a substantially pure population of ASCs or eASCs, e.g. human eASCs. The MSCs, eMSCs, ASCs or eASCs may be stem cells. The composition for use in the present invention may also include cell culture components, e.g., culture media including one or more of amino acids, metals and coenzyme factors. The composition may include small populations of other stromal cells. The composition may also include other non-cellular components which may support the growth and survival of the MSCs under particular circumstances, e.g. implantation, growth in continuous culture, or use as a biomaterial or composition.

The concentration of the MSCs and/or eMSCs in the composition for use in the invention may be at least about 1 x 10⁴ cells/mL, at least about 1 x 10⁵ cells/mL, at least about 1 x 10⁶ cells/mL, at least about 10 x 10⁶ cells/mL, or at least about 40 x 10⁶ cells/mL. Typically the concentration between about 1 x 10⁶ cells/mL and 1 x 10⁷ cells/mL, e.g. between about between about 5 x 10⁶ cells/mL and 1 x 10⁷ cells/mL.

The compositions for use in the invention will generally comprise a pharmaceutically acceptable carrier and/or a diluent. Examples of such carriers and diluents are well known in the art, and may include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations. Typically the compositions for use in the invention comprise DMEM as the carrier, which may optionally be supplemented with serum (e.g. HSA) at a concentration of e.g. up to about 5% , up to about 10%, up to about 15% , up to about 20%, up to about 25% , up to about 30% .

A composition for use in the invention may be sterile and/or fluid to the extent that easy syringability exists. In addition, the composition may be stable under the conditions of manufacture and storage, and/or preserved against the contaminating action of microorganisms such as bacteria and fungi through the use of, for example, parabens, chlorobutanol, phenol, ascorbic acid and thimerosal.

### PREPARATION METHODS

Methods for the isolation and culture of MSCs to provide eMSCs and cell populations, and compositions comprising cell populations are known in the art. Typically methods for the preparation of compositions comprising cell populations comprise the following steps:
(i) isolation of MSCs from tissue and selection by adherence to a suitable surface e.g. plastic
(ii) expansion of MSCs to provide cell populations comprising eMSCs.

Optionally the cell populations for use in the invention may be cryopreserved during and/or subsequent to the expansion step (ii). Optionally the phenotype of the cell populations for use in the invention may be assessed during and/or subsequent to the expansion step (ii). Optionally the cell populations for use in the invention may be isolated subsequent to the expansion step (ii) and resuspended in a pharmaceutically acceptable carrier and/or diluents.

### Isolation of MSC

MSCs for use in the invention may be isolated from any suitable tissue, such as but not limited to peripheral blood, bone marrow, placenta, adipose tissue, periosteum, dental pulp, spleen, pancreas, ligament, skin, tendon, skeletal muscle, umbilical cord and placenta. In a preferred embodiment, the MSCs are obtained from connective tissue, such as the stromal fraction of adipose tissue, hyaline cartilage, bone marrow, skin etc.

In one embodiment, the MSCs are from the stromal fraction of adipose tissue. In another embodiment, the MSCs are obtained from chondrocytes e.g. from hyaline cartilage. In another embodiment, the MSCs are obtained from skin. Also, in another embodiment, the MSCs are obtained from bone marrow.

The MSCs can be obtained from any suitable tissue and from any suitable animal, including humans. Typically, said cells are obtained from post-natal mammalian connective tissues.

The MSCs may also be isolated from any organism of the same or different species as the subject. Any organism with MSCs can be used. In one embodiment the organism may be mammalian, and in another embodiment the organism is human.

Adipose-derived MSCs can be obtained by any means standard in the art. Typically said cells are obtained by disassociating the cells from the source tissue (e.g. lipoaspirate or adipose tissue), typically by treating the tissue with a digestive enzyme such as collagenase. The digested tissue matter is then typically filtered through a filter of between about 20 microns to 1mm. The cells are then isolated (typically by centrifugation) and cultured on an adherent surface (typically tissue culture plates or flasks). Such methods are known in the art and e.g. as disclosed in U.S. Patent No. 6777231. According to this methodology, lipoaspirates are obtained from adipose tissue and the cells derived therefrom. In the course of this methodology, the cells may be washed to remove contaminating debris and red blood cells, preferably with PBS. The cells are digested with collagenase (e.g. at 37°C for 30 minutes, 0.075% collagenase; Type I, Invitrogen, Carlsbad, CA) in PBS. To eliminate remaining red blood cells, the digested sample can be washed (e.g. with 10% fetal bovine serum), treated with 160 mmol/L ClNH4, and finally suspended in DMEM complete medium (DMEM containing 10% FBS, 2 mmol/L glutamine and 1% penicillin/streptomycin). The cells can be filtered through a 40-µm nylon mesh.

The cells are cultured in a suitable tissue culture vessel, comprising a surface suitable for the adherence of MSCs e.g. plastic. Non-adherent cells are removed e.g. by washing in a suitable buffer, to provide an isolated population of adherent stromal cells (e.g. MSC). Cells isolated in this way can be seeded (preferably 2-3x10⁴ cells/cm²) onto tissue culture flasks and expanded at 37°C and 5% CO₂, changing the culture medium every 3-4 days. Cells are preferably passed to a new culture flask (1,000 cells/cm²) when cultures reach 90% of confluence.

Cell isolation is preferably carried out under sterile or GMP conditions.

In certain embodiments, the cells may be cultured for at least about 15 days, at least about 20 days, at least about 25 days, or at least about 30 days. Typically the expansion of cells in culture improves the homogeneity of the cell phenotype in the cell population, such that a substantially pure or homogenous population is obtained.

Cells are preferably detached from the adherent surface (e.g. by means of trypsin) and transferred to a new culture vessel (passaged) when cultures reach about 75%, 80%, 85%, 90% or 95% confluence.

In certain embodiments, the cells are passaged at least three times, which means that the cells are expanded in culture for at least three culture passages. In other embodiments, the cells are passaged at least four times, at least five times, at least six times, at least seven times, at least eight times, at least nine times, or at least ten times.

In certain embodiments, the cells are multiplied in culture for at least three population doublings. In certain embodiments, the cells are expanded in culture for at least four, five, six, seven, eight, nine, ten or 15 population doublings. In certain embodiments, the cells are expanded in culture for less than seven, eight, nine, ten or 15 population doublings. In certain embodiments, the cells are expanded in culture for between about 5 and 10 population doublings.

Cells may be cultured by any technique known in the art for the culturing of stromal stem cells. A discussion of various culture techniques, as well as their scale-up, may be found in Freshney, R.I., Culture of Animal Cells: A Manual of Basic Technique, 4th Edition, Wiley-Liss 2000. Cells may be expanded using culture flasks or bioreactors suitable for large-scale expansion. Bioreactors suitable for the large-scale expansion of mesenchymal stromal cells are commercially available and may include both 2D (i.e. substantially planar) and 3D expansion bioreactors. Examples of such bioreactors include, but are not limited to, a plug flow bioreactor, a perfusion bioreactor, a continuous stirred tank bioreactor, a stationary-bed bioreactor.

In certain embodiments, the cells are cultured by monolayer culture. Any medium capable of supporting MSCs in tissue culture may be used. Media formulations that will support the growth of MSCs include, but are not limited to, Dulbecco's Modified Eagle's Medium (DMEM), alpha modified Minimal Essential Medium (αMEM), and Roswell Park Memorial Institute Media 1640 (RPMI Media 1640). Typically, 0 to 20% Fetal Bovine Serum (FBS) will be added to the above media in order to support the growth of stromal cells. However, a defined medium could be used if the necessary growth factors, cytokines, and hormones in FBS for stromal cells and chondrocytes are identified and provided at appropriate concentrations in the growth medium. Media useful may contain one or more compounds of interest, including, but not limited to antibiotics, mitogenic or differentiating compounds for stromal cells. The cells for use in the invention may be grown at temperatures between 31°C to 37°C in a humidified incubator. The carbon dioxide content may be maintained between 2% to 10% and the oxygen content may be maintained at between 1% and 22%.

Antibiotics which can be added to the medium include, but are not limited to penicillin and streptomycin. The concentration of penicillin in the chemically defined culture medium may be about 10 to about 200 units per ml. The concentration of streptomycin in the chemically defined culture medium may be about 10 to about 200 ug/ml.

Typically, the MSCs as used in the present invention are expanded cell populations, preferably said cells are expanded to provide a substantially pure or homogenous population.

In one embodiment said cell populations are expanded until expression of the marker CD34 is reduced compared to freshly isolated, non-expanded cells. For example the cell population is expanded until expression of the marker CD34 is reduced to a level of less than 50%, less than 35%, less than 30%, or less than 5%, e.g. 35-5%, or 20-10%, of cells in the population. Typically, the cell population is expanded until expression of the marker CD34 is reduced to a level of less 5% of cells in the population. Thus, a population of eMSCs for use in the invention comprises less than 50%, less than 35%, less than 30%, or less than 5%, e.g. 35-5%, or 20-10%, of cells expressing CD34.

In one embodiment said cell populations are expanded until expression of the marker STRO-1 is reduced compared to freshly isolated, non-expanded cells. For example the cell population is expanded until expression of the marker STRO-1 is reduced to a level of less than 50%, less than 35%, less than 30%, or less than 5%, e.g. 35-5%, or 20-10%, of cells in the population. Typically, the cell population is expanded until expression of the marker STRO-1 is reduced to a level of less 5% of cells in the population. Thus, a population of eMSCs for use in the invention comprises less than 50%, less than 35%, less than 30%, or less than 5%, e.g. 35-5%, or 20-10%, of cells expressing STRO-1.

Typically, the cell populations are expanded until expression of the markers CD34 and STRO-1 is reduced compared to freshly isolated, non-expanded cells. For example the cell population is expanded until expression of the markers CD34 and STRO-1 is reduced to a level of less than 50%, less than 35%, less than 30%, or less than 5%, e.g. 35-5%, or 20-10%, of cells in the population. Typically, the cell population is expanded until expression of the markers CD34 and STRO-1 is reduced to a level of less 5% of cells in the population. Thus, a population of eMSCs for use in the invention comprises less than 50%, less than 35%, less than 30%, or less than 5%, e.g. 35-5%, or 20-10%, of cells expressing CD34 and STRO-1.

Expanded MSC populations (e.g. those expressing 5% or less CD34 and/or STRO-1) are advantageous as they present a lower multipotency than freshly isolated cells, i.e. they may have a lower, reduced or no capacity to differentiate into other cell phenotypes as compared to naturally-occurring non-expanded MSC.

It will be apparent to one skilled in the art that the method for preparation of the composition for use in the invention is not limiting, and that compositions for use in the invention prepared in any way are included within the scope of the invention. In one embodiment, the disclosure provides a method of preparing a composition for use in the invention, which comprises: (a) collecting tissue from a donor; (b) obtaining a cell suspension by enzymatic treatment; (c) sedimenting the cell suspension and resuspending the cells in a culture medium; (d) culturing the cells for at least about 5 days or 5 population doublings.

In one embodiment the cells may be cryopreserved prior to administration, e.g. during and/or subsequent to expansion. Thus, the disclosure also provides cryopreserved cells. Methods for cell cryopreservation are known in the art, and typically require the use of suitable cryoprotective agents (e.g. DMSO). Cells may be cryopreserved at any point during the isolation and/or expansion stages and thawed prior to administration. Typically cells may be cryopreserved at passage 3, 4, 5, 6, 7, 8, 9, 10, 12, 15 or higher, e.g. cells may be cryopreserved at passages 3-10 or 5-10. Optionally the cells may be replated and cultured prior to administration.

In one embodiment the cells may be isolated from the cryopreservation and/or culture media and resuspended in a pharmaceutically acceptable carrier and/or diluents prior to administration (e.g. DMEM, optionally supplemented with serum).

In some embodiments, the cell populations for use in the invention may be pre-stimulated in order to enhance one or more of their proliferation capacity, migration capacity, survival capacity, therapeutic effect and inmunoregulatory properties. In some embodiments, pre-stimulation may be achieved by contacting the MSCs with a cytokine. In some embodiments of the invention, pre-stimulation may be achieved by contacting the MSCs with IFN-γ.

In certain embodiments of the invention, the MSCs may be pre-stimulated using a concentration of IFN-γ between 0.1 and 100 ng/ml. In further embodiments, the MSCs may be pre-stimulated using a concentration of IFN-γ between 0.5 and 85 ng/ml, between 1 and 70 ng/ml, between 1.5 and 50 ng/ml, between 2.5 and 40 ng/ml, or between 3 and 30 ng/ml. Pre-stimulation may occur over a stimulation time longer than about 12 hours. Pre-stimulation may occur over a stimulation time longer than about 13 hours, longer than about 18 hours, longer than about 24 hours, longer than about 48 hours, or longer than about 72 hours.

In one embodiment, the MSCs for use in the invention may be stably or transiently transfected or transduced with a nucleic acid of interest using a plasmid, viral or alternative vector strategy. Nucleic acids of interest include, but are not limited to, those encoding gene products which enhance the production of extracellular matrix components found in the tissue type to be repaired, e.g. intestinal wall or vaginal wall.

The transduction of viral vectors carrying regulatory genes into the stromal cells can be performed with viral vectors, including but not limited to adenovirus, retrovirus or adeno-associated virus purified (e.g. by cesium chloride banding) at a multiplicity of infection (viral units:cell) of between about 10:1 to 2000:1. Cells may be exposed to the virus in serum free or serum-containing medium in the absence or presence of a cationic detergent such as polyethyleneimine or Lipofectamine™ for a period of about 1 hour to about 24 hours (Byk T. et al. (1998) Human Gene Therapy 9:2493-2502; Sommer B. et al. (1999) Calcif. Tissue Int. 64:45-49).

Other suitable methods for transferring vectors or plasmids into stromal cells include lipid/DNA complexes, such as those described in U.S. Pat. Nos. 5,578,475; 5,627,175; 5,705,308; 5,744,335; 5,976,567; 6,020,202; and 6,051,429. Suitable reagents include lipofectamine, a 3:1 (w/w) liposome formulation of the poly-cationic lipid 2,3-dioleyloxy-N-[2(sperminecarbox- amido)ethyl]-N,N-dimethyl-1-propanaminium trifluoroacetate (DOSPA) (Chemical Abstracts Registry name: N-[2-(2,5-bis [(3 -aminopropyl)amino] -1-- oxpentyl}amino)ethyl]-N,N-dimethyl-2,3 -bis(9-octadecenyloxy)-1 - propanamin- ium trifluoroacetate), and the neutral lipid dioleoyl phosphatidylethanolamine (DOPE) in membrane filtered water. Exemplary is the formulation Lipofectamine 2000TM (available from Gibco/Life Technologies # 11668019). Other reagents include: FuGENETM 6 Transfection Reagent (a blend of lipids in non-liposomal form and other compounds in 80% ethanol, obtainable from Roche Diagnostics Corp. # 1814443); and LipoTAXITM transfection reagent (a lipid formulation from Invitrogen Corp., #204110). Transfection of stromal cells can be performed by electroporation, e.g., as described in M.L. Roach and J.D. McNeish (2002) Methods in Mol. Biol. 185:1. Suitable viral vector systems for producing stromal cells with stable genetic alterations may be based on adenoviruses and retroviruses, and may be prepared using commercially available virus components.

The transfection of plasmid vectors carrying regulatory genes into the MSCs can be achieved in monolayer cultures by the use of calcium phosphate DNA precipitation or cationic detergent methods (Lipofectamine™, DOTAP) or in three dimensional cultures by the incorporation of the plasmid DNA vectors directly into the biocompatible polymer (Bonadio J. et al. (1999) Nat. Med. 5:753-759).

For the tracking and detection of functional proteins encoded by these genes, the viral or plasmid DNA vectors may contain a readily detectable marker gene, such as the green fluorescent protein or beta-galactosidase enzyme, both of which can be tracked by histochemical means.

Subsequent to expansion it is preferred that cell populations for use in the invention are assayed to determine the expression of characteristic markers to confirm their phenotype, which can be carried out by using conventional means.

The term "expressed" is used to describe the presence of a marker within a cell. In order to be considered as being expressed, a marker must be present at a detectable level. By "detectable level" is meant that the marker can be detected using one of the standard laboratory methodologies such as PCR, blotting or FACS analysis. The phenotypic surface marker characterization of a population of MSCs may be performed by any method known in the art. By way of example, but not limitation, this phenotypic characterization may be performed by individual cell staining. Such staining may be achieved through the use of antibodies. This may be direct staining, by using a labeled antibody or indirect staining, using a second labeled antibody against a primary antibody specific for the cell marker. Antibody binding may be detected by any method known in the art. Antibody binding may also be detected by flow cytometry, immunofluorescence microscopy or radiography.

Alternatively or additionally, a gene is considered to be expressed by a cell of the population, if expression can be reasonably detected after 30 PCR cycles, which corresponds to an expression level in the cell of at least about 100 copies per cell. The terms "express" and "expression" have corresponding meanings. At an expression level below this threshold, a marker is considered not to be expressed. The comparison between the expression level of a marker in an adult stromal cell, and the expression level of the same marker in another cell, such as for example an embryonic stem cell, may preferably be conducted by comparing the two cell types that have been isolated from the same species. Preferably this species is a mammal, and more preferably this species is human. Such comparison may conveniently be conducted using a reverse transcriptase polymerase chain reaction (RT-PCR) experiment.

Cell-surface markers can be identified by any suitable conventional technique, usually based on a positive/negative selection; for example, monoclonal antibodies against cell-surface markers, whose presence/absence in the cells is to be confirmed, can be used; although other techniques can also be used. Thus, in a particular embodiment, monoclonal antibodies against one, two, three, four, five, six, seven of or all of CD9, CD10, CD13, CD29, CD44, CD49A, CD51, CD54, CD55, CD58, CD59, CD90 and CD105 are used in order to confirm the absence of said markers in the selected cells; and monoclonal antibodies against one, two, three, four, of or all of Factor VIII, alpha-actin, desmin, S-100, keratin ,CD11b, CD11c, CD14, CD45, HLAII, CD31, CD34, CD45, STRO-1 and CD133 are used in order to confirm the presence thereof or detectable expression levels of, at least one of and preferably all of, said markers.

In a further embodiment monoclonal antibodies against at least one, two, three or all of CD34; CD45; CD31; CD14 e.g. CD31 and/or CD34 are used in order to confirm the presence or detectable expression levels of said markers. In a further embodiment, monoclonal antibodies against CD34 are used in order to confirm the absence of said marker in the selected cells. In a further embodiment, monoclonal antibodies against STRO-1 is used in order to confirm the absence of said marker in the selected cells. In a further embodiment monoclonal antibodies against at least one, two, three or all of CD29; CD59; CD90; CD105 e.g. CD59 and/or CD90 are used in order to confirm the presence or detectable expression levels of thereof.

Said monoclonal antibodies are known, commercially available or can be obtained by a skilled person in the art by conventional methods.

IFN-γ-inducible IDO activity in the selected cells can be determined by any suitable conventional assay. For example, the selected cells can be stimulated with IFN-γ and assayed for IDO expression; then conventional Western-blot analysis for IDO protein expression can be performed and IDO enzyme activity following IFN-γ stimulation of the selected cells can be measured by tryptophan-to-kynurenine conversion with for example via High Performance Liquid Chromatography (HPLC) analysis and photometric determination of kynurenine concentration in the supernatant as the readout. Since the cells for use in the invention express IDO under certain conditions, any suitable technique which allows the detection of IDO activity following IFN-γ stimulation may be used for selecting the cells for use in the invention. A suitable assay for determining IFN-γ-inducible IDO activity in the selected cells is disclosed in WO2007039150. The amount of IDO produced depends on the number of cells per square centimetre, which is preferably at a level of 5000cells/cm2 or more, but not limited to this concentration and the concentration of IFN- γ, which ideally is 3ng/ml or more, but not limited to this concentration. The activity of IDO produced under the described conditions will result in a detectable production of kynurenine in the µM range after 24hours or more.

### ADMINISTRATION

In one embodiment, a composition for use in the invention may be prepared for systemic administration (*e.g*. rectally, nasally, buccally, vaginally, via an implanted reservoir or via inhalation). In another embodiment, a composition for use in the invention may be prepared for local administration. A composition for use in the invention may be administered by the parenteral route. A composition may be administered by the subcutaneous, intracutaneous, intravenous, intramuscular, intra articular, intrasynovial, intrasternal, intrathecal, intralesional, intralymphatic and intracranial routes. In one embodiment, the MSCs are administered by the intravenous route, for example by intravenous injection. Alternatively, the MSCs are administered via the intralymphatic route, for example by intralymphatic injection, *e.g*. by intralymphatic injection to a lymphatic organs such as a peripheral lymphatic organ, including but not limited to the lymph nodes, most preferably an axillary or inguinal lymph node. In each of these embodiments, the MSCs can be ASCs, for example eASCs. As used herein, the term "lymphatic system" is to be given its usual meaning in the art and refers to lymphoid tissue, such as a lymphatic organ, connected by a conducting system of lymph vessels and lymph capillaries. The term "lymphatic organ" refers to lymph nodes, most preferably an axillary or inguinal lymph node.

Typically, the patient is a human.

Thus, in one embodiment, the invention provides ASCs for use in treating moderate rheumatoid arthritis (CDAI score of >10 to ≤22, and optionally having a DAS28 score of >3.2 to ≤5.1 and/or a RAPID3 score of >6 to ≤12), in a human subject wherein the ASCs are administered intralymphatically, for example by intralymphatic administration and wherein the subject is first treated at no more than about 12 months from first diagnosis or onset of disease symptoms. In another embodiment, the invention provides ASCs for use in treating rheumatoid arthritis, preferably moderate rheumatoid arthritis (CDAI score of >10 to ≤22, and optionally having a DAS28 score of >3.2 to ≤5.1 and/or a RAPID3 score of >6 to ≤12), in a human subject wherein the ASCs are administered intravenously, for example by intravenous injection and wherein the subject is first treated at no more than about 12 months from first diagnosis or onset of disease symptoms. The ASCs can optionally be administered repeatedly with an interval of at least 1 day.

In one embodiment, the MSCs used in the invention may be autologous with respect to the subject to be treated. In a further embodiment, the MSCs used in the invention may be allogeneic or xenogeneic with respect to the subject to be treated. Allogenic MSCs derived from a donor may theoretically be used for the treatment of any patient, irrespective of MHC incompatibility. In one embodiment, the composition for use in the invention may be administered by injection or implantation of the composition at one or more target sites in the subject to be treated. In a further embodiment, the composition for use in the invention may be inserted into a delivery device which facilitates introduction of the composition into the subject by injection or implantation. In one embodiment the delivery device may comprise a catheter. In a further embodiment, the delivery device may comprise a syringe.

### DOSAGE

The dosage of MSCs and any further therapeutic agent will vary depending on the symptoms, age and body weight of the patient, the nature and severity of the disorder to be treated or prevented, the route of administration, and the form of the further therapeutic agent. The compositions for use in the invention may be administered in a single dose or in divided doses. Appropriate dosages for MSCs and any further therapeutic agent(s) may be determined by known techniques.

Typically said dose is about 10 x 10⁶ cells/kg of subject weight or lower, is about 9 x 10⁶ cells/kg or lower, is about 8 x 10⁶ cells/kg or lower, is about 7 x 10⁶ cells/kg or lower, is about 6 x 10⁶ cells/kg or lower, is about 5 x 10⁶ cells/kg or lower. In an alternative embodiment said dose may be between about 0.25 x 10⁶ cells/kg to about 5 x 10⁶ cells/kg; or more preferably about 1 x 10⁶ cells/kg to about 5 x 10⁶ cells/kg. Accordingly in further alternative embodiments the dose may be about 0.25 x 10⁶ cells/kg, 0.5 x 10⁶ cells/kg, 0.6 x 10⁶ cells/kg, 0.7 x 10⁶ cells/kg; 0.8 x 10⁶ cells/kg; 0.9 x 10⁶ cells/kg; 1.1 x 10⁶ cells/kg; 1.2 x 10⁶ cells/kg; 1.3 x 10⁶ cells/kg; 1.4 x 10⁶ cells/kg; 1.5 x 10⁶ cells/kg; 1.6 x 10⁶ cells/kg; 1.7 x 10⁶ cells/kg; 1.8 x 10⁶ cells/kg; 1.9 x 10⁶ cells/kg or 2 x 10⁶ cells/kg. The dose may, in other embodiments, be between 0.1 and 1 million cells / kg; or between 1 and 2 million cells / kg; or between 2 and 3 million cells / kg; or between 3 and 4 million cells / kg; or between 4 and 5 million cells / kg; or between 5 and 6 million cells / kg; or between 6 and 7 million cells / kg; or between 7 and 8 million cells / kg; or between 8 and 9 million cells / kg; or between 9 and 10 million cells / kg.

In an alternative embodiment the cells may be administered to the patient as a fixed dose, independent of patient weight. Typically said dose is between about 10 million cells and 500 million cells, e.g. said dose is about 10 x 10⁶ cells, 50 x 10⁶ cells, 10 x 10⁷ cells, 50 x 10⁷ cells.

In one embodiment the invention provides MSCs, such as ASCs, for use in treating moderate rheumatoid arthritis having a CDAI score of >10 to ≤22, in a human subject wherein the MSCs are administered by an intralymphatic route, for example by intralymphatic injection, at a dose of between 0.05 and 0.25 million cells/kg of subject weight, between 0.1 and 1 million cells/kg of subject weight and wherein the subject is first treated at no more than about 12 months from first diagnosis or onset of disease symptoms. In another embodiment, a fixed dose of between about 0.1 and 5 million cells, or about 5 million cells, or about 10 million cells is administered by an intralymphatic route, for example by intralymphatic injection.

In another embodiment the invention provides MSCs, such as ASCs, for use in treating moderate rheumatoid arthritis having a CDAI score of >10 to ≤22, in a human subject wherein the MSCs are administered by an intravenous route, for example by intravenous injection, at a dose of between about 0.25 x 10⁶ cells/kg to about 10 x 10⁶ cells/kg of subject weight, for example between about 0.25 x 10⁶ cells/kg to about 5 x 10⁶ cells/kg of subject weight and wherein the subject is first treated at no more than about 12 months from first diagnosis or onset of disease symptoms. A typical dose is 4 x 10⁶ cells/kg of subject weight. 8 x 10⁶ cells/kg of subject weight may also be used.

The precise time of administration and amount of any particular agent that will yield the most effective treatment in a given patient will depend upon the activity, pharmacokinetics, and bioavailability of the agent, the physiological condition of the patient (including age, sex, disease type and stage, general physical condition, responsiveness to a given dosage and type of medication), the route of administration, *etc..* The information presented herein may be used to optimize the treatment, e.g., determining the optimum time and/or amount of administration, which will require no more than routine experimentation, such as monitoring the subject and adjusting the dosage and/or timing. While the subject is being treated, the health of the subject may be monitored by measuring one or more of relevant indices at predetermined times during a 24-hour period. Treatment regimens, including dosages, times of administration and formulations, may be optimized according to the results of such monitoring.

Treatment may be initiated with smaller dosages which are less than the optimum dose. Thereafter, the dosage may be increased by small increments until the optimum therapeutic effect is attained.

The combined use of several therapeutic agents may reduce the required dosage for any individual component because the onset and duration of effect of the different components may be complimentary. In such combined therapy, the different active agents may be delivered together or separately, and simultaneously or at different times within the day.

### FURTHER THERAPEUTIC AGENTS

In one embodiment, the pharmaceutical composition for use in the invention may contain or alternatively may be administered in conjunction with one or more (or two or more, or three or more, e.g. 1, 2, 3, 4 or 5) further therapeutic agents.

In some embodiments, the MSCs and the one or more further therapeutic agents may be administered to the subject simultaneously. In other embodiments, the MSCs and the one or more further therapeutic agents may be administered to the subject sequentially. The one or more further therapeutic agents may be administered before or after administration of the MSCs.

Said further therapeutic agent may be selected from the following: an analgesic, such as a nonsteroidal anti-inflammatory drug, an opiate agonist or a salicylate; an anti-infective agent, such as an antihelmintic, an antianaerobic, an antibiotic, an aminoglycoside antibiotic, an antifungal antibiotic, a cephalosporin antibiotic, a macrolide antibiotic, a β-lactam antibiotic, a penicillin antibiotic, a quinolone antibiotic, a sulfonamide antibiotic, a tetracycline antibiotic, an antimycobacterial, an antituberculosis antimycobacterial, an antiprotozoal, an antimalarial antiprotozoal, an antiviral agent, an anti-retroviral agent, a scabicide, an anti-inflammatory agent, a corticosteroid anti-inflammatory agent, an antipruritics/local anesthetic, a topical anti-infective, an antifungal topical anti-infective, an antiviral topical anti-infective; an electrolytic and renal agent, such as an acidifying agent, an alkalinizing agent, a diuretic, a carbonic anhydrase inhibitor diuretic, a loop diuretic, an osmotic diuretic, a potassium-sparing diuretic, a thiazide diuretic, an electrolyte replacement, and an uricosuric agent; an enzyme, such as a pancreatic enzyme and a thrombolytic enzyme; a gastrointestinal agent, such as an antidiarrheal, an antiemetic, a gastrointestinal anti-inflammatory agent, a salicylate gastrointestinal anti-inflammatory agent, an antacid anti-ulcer agent, a gastric acid-pump inhibitor anti-ulcer agent, a gastric mucosal anti-ulcer agent, a H2-blocker anti-ulcer agent, a cholelitholytic agent, a digestant, an emetic, a laxative and stool softener, and a prokinetic agent; a general anesthetic, such as an inhalation anesthetic, a halogenated inhalation anesthetic, an intravenous anesthetic, a barbiturate intravenous anesthetic, a benzodiazepine intravenous anesthetic, and an opiate agonist intravenous anesthetic; a hormone or hormone modifier, such as an abortifacient, an adrenal agent, a corticosteroid adrenal agent, an androgen, an anti-androgen, an immunobiologic agent, such as an immunoglobulin, an immunosuppressive, a toxoid, and a vaccine; a local anesthetic, such as an amide local anesthetic and an ester local anesthetic; a musculoskeletal agent, such as an anti-gout anti-inflammatory agent, a corticosteroid anti-inflammatory agent, a gold compound anti-inflammatory agent, an immunosuppressive anti-inflammatory agent, a non-steroidal anti-inflammatory drug (NSAID), a salicylate anti-inflammatory agent, a mineral; and a vitamins, such as vitamin A, vitamin B, vitamin C, vitamin D, vitamin E, and vitamin K.

Said further therapeutic agent preferably comprises one or more of disease-modifying antirheumatic drugs (DMARDs), non-steroidal anti-inflammatories (NSAIDs), biological medicinal products and corticosteroids. NSAIDs include but are not limited to Cox-2 inhibitors, diclofenac, ibuprofen, naproxen, celecoxib, mefenamic acid, etoricoxib, indometacin and aspirin. Corticosteroids include, but are not limited to, triamcinolone, cortisone, prednisone, and methylprednisolone. Disease-modifying antirheumatic drugs (DMARDs), include, but are not limited to, auranofin, choroquine, cyclosporine, cyclophosphamide, gold preparations, hydroxychloroquine sulphate, leflunomide, methotrexate, penicillamine, sodium aurothiomalate, sulfasalazine.

In one embodiment the patients may additionally be treated with at least 2 or 3 DMARDs, preferably selected from the group consisting of hydroxychloroquine, leflunomide, methotrexate, minocycline, and sulfasalazine (e.g., methotrexate and hydroxychloroquine; methotrexate and leflunomide; methotrexate and sulfasalazine; sulfasalazine and hydroxychloroquine; methotrexate, hydroxychloroquine and sulfasalazine).

Optionally the patients may additionally be treated with biological medicinal products. Biological medicinal products, include but are not limited to selective costimulation modulators, TNF-α inhibitors, IL-1 inhibitors, IL-6 inhibitors & B-cell targeted therapies. Typically, the patient may additionally be treated with at least one biological treatment, for example a TNF-α inhibitor. Accordingly, in one aspect of the invention the patient may additionally be treated with at least one biological treatment which is a TNF-α inhibitor, typically Adalimumab (Humira), Certolizumab (Cimzia), Etanercept (Enbrel), Golimumab (Simponi), and/or Infliximab (Remicade).

In another embodiment, the further therapeutic agent may be a growth factor or other molecule that affects cell proliferation or activation. In a further embodiment that growth factor may induce final differentiation. In another embodiment, the growth factor may be a variant or fragment of a naturally-occurring growth factor. Methods of producing such variants are well known in the art, and may include, for example, making conservative amino acid changes, or by mutagenesis and assaying the resulting variant for the required functionality.

The invention will now be further illustrated by the following examples. These examples are provided by way of illustration only, and are not intended to be limiting.

### EXAMPLE 1: Early treatment with ASCs attenuates the evolution of arthritis

On the day of commencement of the study, each mouse (young, healthy, male DBA/1 (H-2^{q}) 8 weeks old mice) was injected subcutaneously in the tail (2-3cm from body) with a dose of an emulsion of Chicken Collagen Type II (1mg/ml final concentration) in Complete Freund's Adjuvant (Mycobacterium Tuberculosis 2mg/ml final concentration) in a volume of 0.1ml/animal.

The distribution of animals to each experimental group was made in a randomized manner at the start of the study. Treatment was performed at day 15, 18 and 21 after collagen injection (n=12 mice per group). Arthritis score was monitored from day 21. Adipose tissue derived stem cells (ASCs) were obtained from human lipoaspirates (see Example 7) and administered (200.000 cells) as a suspension in Ringer Lactate solution (vehicle) via the intravenous route (tail vein).

The Arthritis Index Score was assessed for each animal from day 21 (from collagen injection) and until the end of the study. The severity of the arthritis was scored in both front and hind paws according to the following arthritis index scoring system. The final score is the sum of the scores for the 4 paws. The maximum score is 16.

| **SCORE** | **SIGN OF ARTHRITIS** |
|---|---|
| 0 | No signs of arthritis |
| 1 | Swelling and/or reddening of the paw or 1 digit. |
| 2 | Two groups of joints inflamed (swelling and/or reddening) |
| 3 | More than two groups of joints inflamed (swelling and/or reddening) |
| 4 | Inflammation of the whole paw. Severe arthritis |

### Experimental groups:

A) CIA, no treatment (n=12)
B) CIA, treated with ASCs intravenously (200.000 cells) at d15, d18, d21 (n=12)
As seen in Figure 1, treatment with ASCs of mice at early stages of arthritis significantly attenuated the course (Figure 1A) and incidence (Figure 1B) of the disease.

### EXAMPLE 2: Extended treatment with ASCs prolongs therapeutic effect in early arthritis

On the day of commencement of the study, each mouse (young, healthy, male DBA/1 (H-2^{q}) 8 weeks old mice were) was injected subcutaneously in the tail (2-3cm from body) with the first dose of an emulsion of Chicken Collagen Type II (1mg/ml final concentration) in Complete Freund's Adjuvant (Mycobacterium Tuberculosis lmg/ml final concentration) in a volume of 0.1ml/animal. Afterwards, 21 days after the first injection of collagen, a second injection (booster) of type II collagen (0.1mls/animal) was administered to each animal, again subcutaneously in the tail but at a different location from the first injection. In this occasion the collagen suspension was made using Incomplete Freund's Adjuvant (no M. Tuberculosis).

In this study, treatment with ASCs was started when early stages of arthritis were already established (arthritis index score of 2-4). In order to ensure homogeneity of the arthritis index score in each experimental group, the distribution of animals to each experimental group was not made in a randomized manner at the start of the study. Instead, animals were assigned to experimental groups as they attained an arthritis index score between 2 and 4 (n=12 per group). Arthritis score was monitored as indicated above.

### Experimental groups:

A) CIA, no treatment (n=12)
B) CIA, treated with ASCs intravenously (200.000 cells) at d1, d3, d5 (n=12)
C) CIA, treated with ASCs intravenously (200.000 cells) at d1, d3, d5, d8, d15, d22, d29 (n=12)

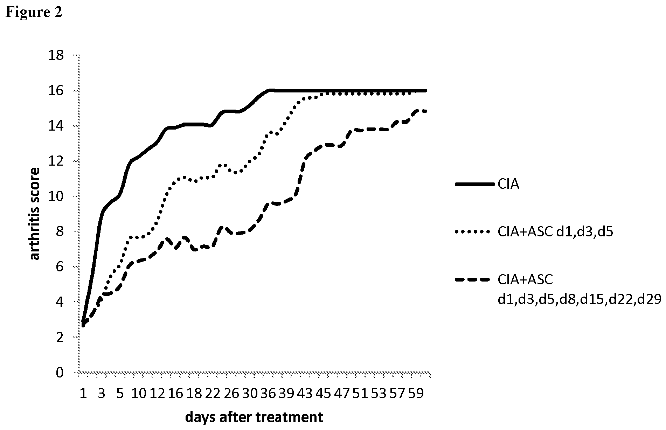

As shown in Figure 2, treatment with ASCs at d1, d3 and d5 reduced the severity of the disease. Notably, the extended treatment (at d8, d15, d22 and d29) enhanced and prolonged the therapeutic effect of the cells.

### EXAMPLE 3: Dose and timing optimization of ASC treatment of early arthritis

### Experimental groups:

A) Healthy (n=5)
B) CIA, no treatment (n=10)
C) CIA, treated with cultured ASCs intravenously (1.000.000 cells) at d1, d3, d5. (n=11)
D) CIA, treated with cultured ASCs intravenously (200.000 cells) at d1, d3, d5. (n=11)
E) CIA, treated with cultured ASCs intravenously (40.000 cells) at d1, d3, d5. (n=11)
F) CIA, treated with cultured ASCs intravenously (8.000 cells) at d1, d3, d5. (n=11)
G) CIA, treated with cultured ASCs intravenously (1.000.000 cells) at d1, d8, d15. (n=11)
H) CIA, treated with cultured ASCs intravenously (200.000 cells) at d1, d8, d15. (n=11)
I) CIA, treated with cultured ASCs intravenously (40.000 cells) at d1, d8, d15. (n=11)
J) CIA, treated with cultured ASCs intravenously (8.000 cells) at d1, d8, d15. (n=11)

Young, healthy, male DBA/1 (H-2^{q}) 8 weeks old mice were used in the present study. CIA was generated as indicated for Example 2 and treatments were started once mice showed early stages of the disease (arthritis score 2-4).

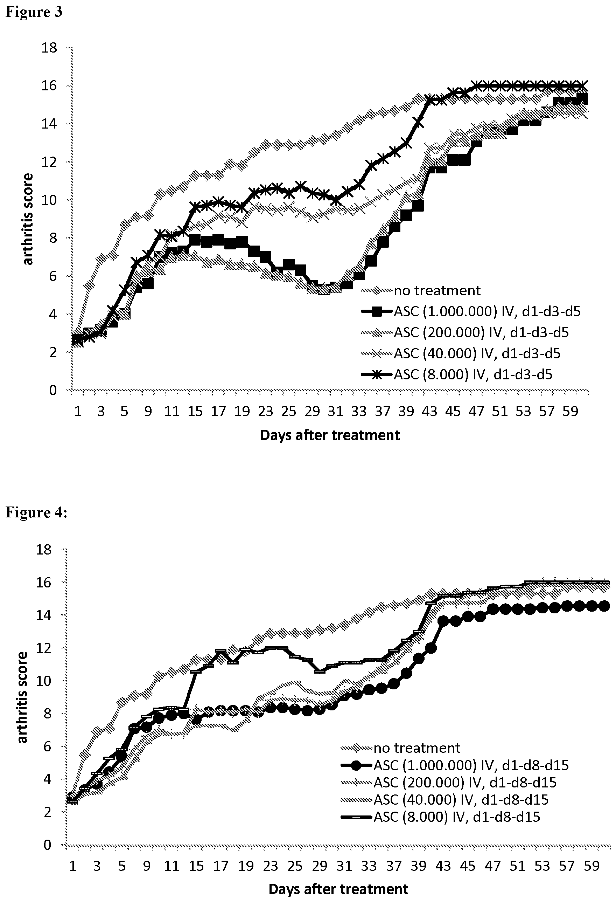

As shown in Figures 3 and 4, three intravenous administrations with ASCs with a day interval at d1, d3 and d5 (Figure 3) or with a week interval at d1, d8 and d15 (Figure 4) reduced the severity of arthritis. Doses between 1 million and 0.04 million cells were particularly effective.

### EXAMPLE 4: Treatment with ASCs reduces pro-inflammatory cytokines in serum and increases T cells with regulatory phenotype in peripheral blood

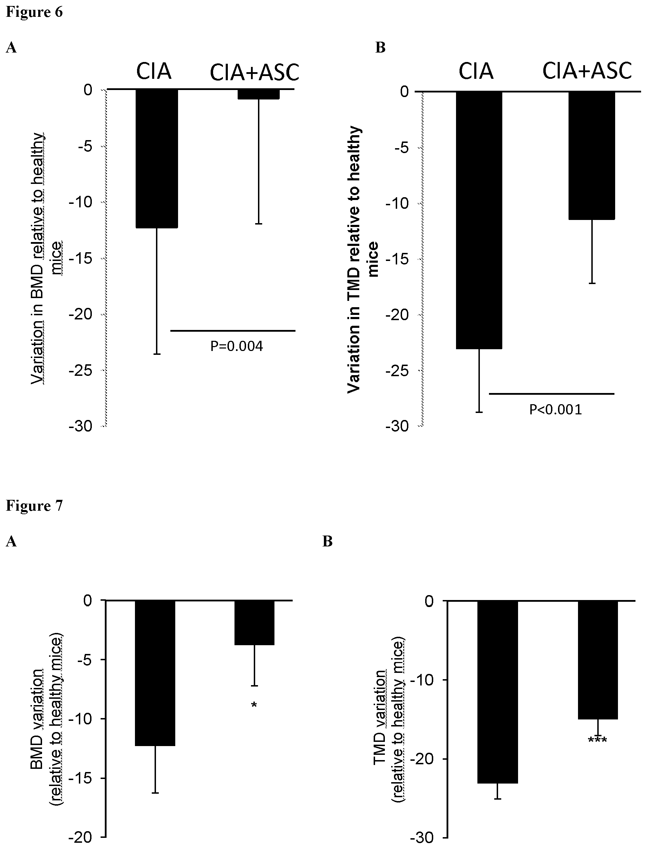

At day 22 after the beginning of treatment (mice treated intravenously with 200.000 ASCs at day 1, day 8 and day 15), peripheral blood was collected and pro-inflammatory cytokines were determined in the plasma and the regulatory T cell population was measured in the cell fraction of the blood. As shown in Figure 5, treatment with ASCs reduced levels of IL6 (Fig. 5A), IL17 (Fig. 5B) and IL23 (Fig. 5C) and increased levels of regulatory T cells (Fig. 5D). Moreover, treatment with ASCs also reduced the arthritis-mediated joint damage after 50 days of the initiation of the treatment, as determined (MicroCT scanning) by significant reduction of the bone (Figure 6A) and trabecular mineral density loss (Figure 6B) compared to healthy mice.

### EXAMPLE 5: Intralymphatic administration of ASCs at early stages of arthritis reduce arthritis score, paw edema and joint damage

Young, healthy, male DBA/1 (H-2^{q}) 8 weeks old mice were used in the study. CIA was generated as indicated for Example 2 and treatments were started once mice showed early stages of the disease (arthritis score 2-4).

### Experimental groups:

A) CIA, no treatment (n=12)
B) CIA, treated with ASCs intralymphatically (48.500 cells in each of right and left inguinal lymph nodes) at d1, d8, dl5 (n=12)
In addition to arthritis score, hind paw edema was also monitored (using a plethysmometer). As shown in Figure 8, intralymphatic administration of ASCs at early stages of arthritis reduced the evolution of the disease as indicated by the reduction of the mean arthritis score of the groups (Fig. 8A and B) and the paw volume (Fig. 8C and D). The mean arthritis score is the mean of the clinical score in the whole study.

Moreover, intralymphatic administration of ASCs also reduced the joint damage (bone and trabecular mineral density loss compared to healthy mice) at day 50, as shown in Figure 7.

### EXAMPLE 6: The effects of ASCs administered intralymphatically are mediated by the induction of T cells with regulatory phenotype (CD4+CD25+FoxP3+) and T cells with anti-inflammatory phenotype (CD4+IL10+ cells)

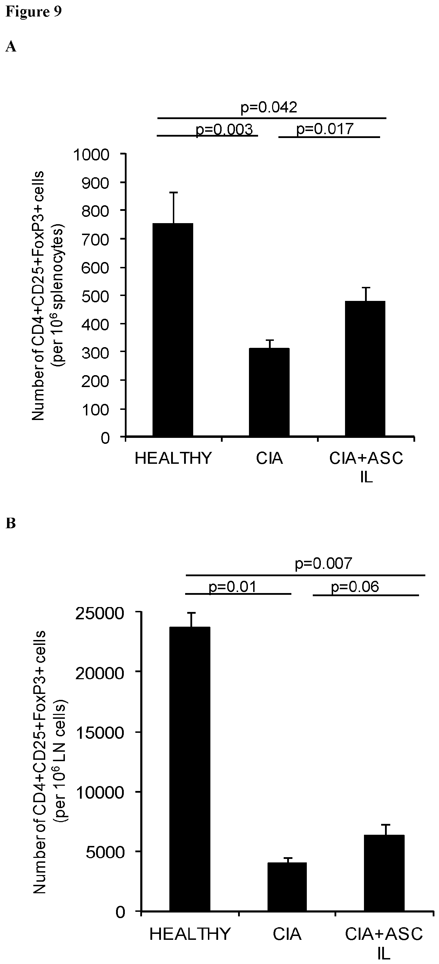

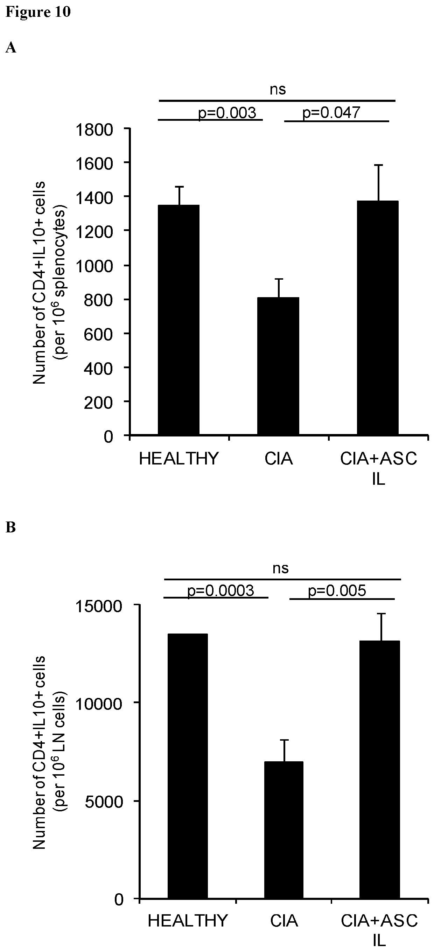

Arthritis (CIA) was induced and mice were treated intralymphatically with ASCs as described in Example 5. After 22 days of the beginning of the treatment, mice were sacrificed and the levels of CD4+CD25+ T cells expressing FoxP3 (regulatory T cells) and CD4 T cells expressing the anti-inflammatory cytokine IL10 were determined in the spleen and lymph nodes. As shown in Figure 9, the level of CD4+CD25+ T cells expressing FoxP3 is increased in the spleen (Fig. 9A) and lymph node (Fig. 9B) of ASC-treated mice, compared to untreated mice. As shown in Figure 10, the level of CD4 T cells expressing IL10 is also increased in the spleen (Fig. 10A) and lymph node (Fig. 10B) of ASC-treated mice, compared to untreated mice.

These examples show that treatment with ASCs within a particular time window (at early stages of the disease, when the disease is moderate), is surprisingly effective. Particularly good results can be achieved when the ASCs are administered at intervals, and/or via the intralymphatic route.

### EXAMPLE 7: Administration of ASCs is particularly effective in human patients with early/moderate rheumatoid arthritis.

This was a multicentre, dose escalation, randomised, single-blind, placebo-controlled phase Ib/IIa clinical trial, with a follow-up period of up to 6months (EudraCT no.: 2010-021602-37; clinicaltrials.gov number: NCT01663116).

The objectives were to evaluate the safety and tolerability of the intravenous administration of allogeneic expanded adipose-derived stem cells (eASCs) in refractory rheumatoid arthritis (RA) patients, as well as to obtain preliminary clinical activity data in this population.

### eASCs

Lipoaspirates were subjected to digestion with collagenase, after extensive washing. Then, red blood cells were removed by erythrocyte lysis, and the stromal vascular fraction was obtained through filtration. ASCs were isolated, and in vitro expansion was carried out through successive expansion passages. Finally, eASCs were subjected to a cryopreservation process to obtain a master cell bank from which a new expansion process was carried out to obtain working cell banks, that were also cryopreserved. Batches for clinical use were obtained after thawing cells from the working cell banks, recovering the cells in adequate culture conditions and formulating them in the corresponding administration vehicle. At various stages during the process the eASCs were tested for number, viability, population doublings, morphology, potency, identity, purity, sterility, mycoplasmas and genetic stability, amongst other quality controls. The product for clinical use was released after confirmation of compliance with the established quality specifications.

### Patients

Given the lack of safety and efficacy data of intravenously administered eASCs or other mesenchymal stem cells in patients with RA at the time of study design, the sample size was based on other mesenchymal stem cells clinical trials for other indications.

Eligible patients were adults with a diagnosis of RA for ≥6 months, treated with at least one non-biologic agent, and who had shown previous failure to treatment with at least two biologies. Their EULAR Disease Activity Score (DAS28-ESR) had to be >3.2; they had to have 4 tender joints to palpation and 4 swollen joints (based on a 68/66-joint count) and had to be receiving treatment on an outpatient basis.

Eighteen investigational sites recruited a total of 67 patients. Fourteen patients were screening failures, therefore 53 patients continued in the trial and received at least one dose of the study treatment (ITT population). Major protocol violations occurred in 5 patients, therefore the PP population consisted of 48 patients. Ten patients discontinued the study prematurely, 4 in cohort A, 1 in cohort B, 2 in cohort C and 3 in placebo group.

53 patients with active refractory RA (failure to at least two biologicals) were randomised to receive three intravenous infusions (iv) of eASCs: 1 million/kg (cohort A; 20 patients), 2 million/kg (cohort B; 6 patients), 4 million/kg (cohort C; 6 patients), or placebo (7 patients) administered at days 1, 8 and 15, and they were followed for therapy assessment for 24 weeks. Background non-biologic DMARDs were kept stable, as well as NSAIDs and glucocorticoids (≤10 mg per day of prednisone or equivalent). Rescue therapy with any DMARD, including biologics was allowed after the 3^{rd} month. Follow-up visits were conducted at weeks 1, 2 and 3 (visits 1-3), and at months 1, 2, 3, 4, 5 and 6 (visits 4-9). In order to safeguard patients' safety as much as possible, safety assessments were single-blinded (blinded patient and unblinded investigator), whereas efficacy assessments were double-blinded. This study was performed according to the applicable regulations, according to GCP standards (CPMP/ICH/135/95) and to the amended Declaration of Helsinki, (Seoul, October 2008). after approval by the corresponding ethics committees. Patients provided written informed consent.

Baseline demographic and clinical characteristics were typical of refractory RA and generally comparable among treatment groups (with the exception in the number of previous DMARDs, which was higher in cohort C compared to the other groups). The mean (SD) number of previous biologics was 2.92 (1.44).

### Statistical analysis

A descriptive analysis, including anthropometric data, variables related to the medical history of patients, efficacy endpoints reported at baseline and baseline laboratory parameters, was conducted. Analysis of the primary safety endpoint was performed on the intent-to-treat (ITT) population. Number and percentages of patients who experienced AEs, SAEs, treatment-related AEs and treatment-related SAEs were described for the overall population and by treatment group, as well as those who reported grade 3-4 AEs. These values might be compared between the groups in the maintenance phase using a χ² test; otherwise, a Fisher's exact test was used. Laboratory parameters, particularly of the parameters included in the selection criteria, were described by visit.

Analysis of the secondary efficacy endpoints was performed on the ITT and per-protocol (PP) populations. ACR and EULAR responses at week 24 were compared between groups using a Cochran-Mantel-Haenszel χ² test. An analysis of covariance (ANCOVA) was used to compare the main efficacy endpoints between the treatment groups, including basal value in the model as covariate. Statistical analysis was conducted with SAS package v9.2.

### Clinical efficacy

### • Moderate RA

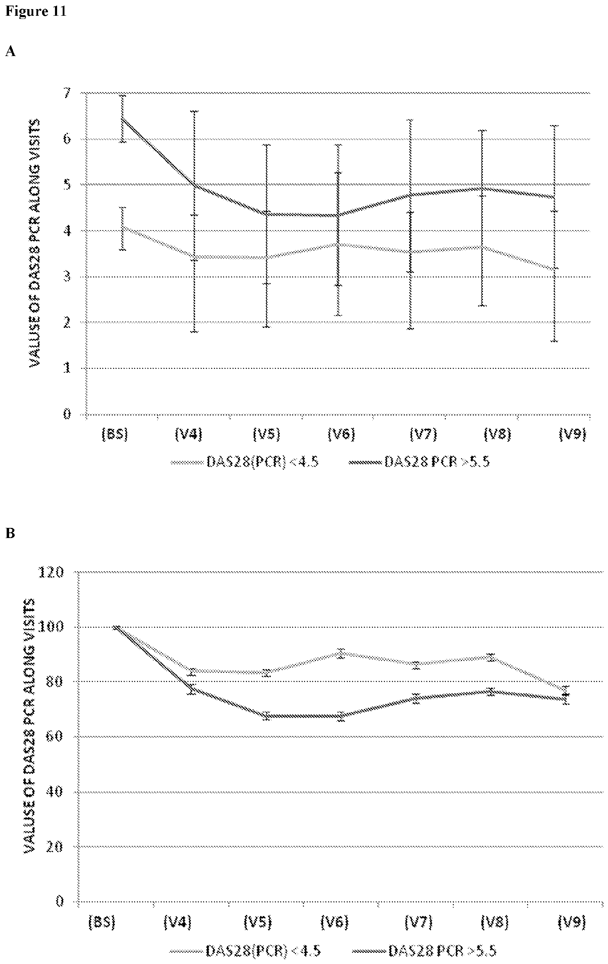

Patients were divided into a population with moderate rheumatoid arthritis (DAS28(PCR) values between 3.2 and 4.5) and a population with severe rheumatoid arthritis (DAS28(PCR) values over 5.5) at baseline. In the moderate RA population, the patient evolution after three intravenous administrations of eASCs was reduced to levels below 4 and controlled during the 6 months of the treatment. However, in the group of patients that presented with severe rheumatoid arthritis the reduction of the disease is less controlled at later stages, after visit 5 (Figure 11A).

Figure 11B shows normalized values of DAS28 considering baseline as 100%. The data show that patients with low levels of DAS28 do present a lower reduction of the DAS28 levels in the first three months compared with the group of patients with high DAS28 levels.

In the case of the CDAI score, the group of patients with low CDAI score maintained a reduced score across visits, whereas patients with a high score did not reach low levels. Figure 12A represents the mean of a group of 8 patients with CDAI score below 22 at baseline (BS) and 6 months after treatment (visits 4, 5, 6, 7, 8, and 9) and a group of 26 patients with CDAI score between 22 and 40 at baseline and 6 months after treatment (visits 4, 5, 6, 7, 8, and 9).

Figure 12B represents normalized values considering baseline as 100% of the score. The data show that patients with low levels of CDAI (<22 at baseline) do present a much higher reduction of the CDAI levels compared with the group of patients with high CDAI levels (>22 and <40 at baseline).

### • ACR20/50/70 responses

Secondary exploratory efficacy endpoints included the proportion of ACR20, ACR50 and ACR70 patients and EULAR response (DAS28-ESR and DAS28-CRP). Other efficacy outcomes considered were the Short Form 36 Health Survey (SF-36) questionnaire, and the RA MRI score (RAMRIS).

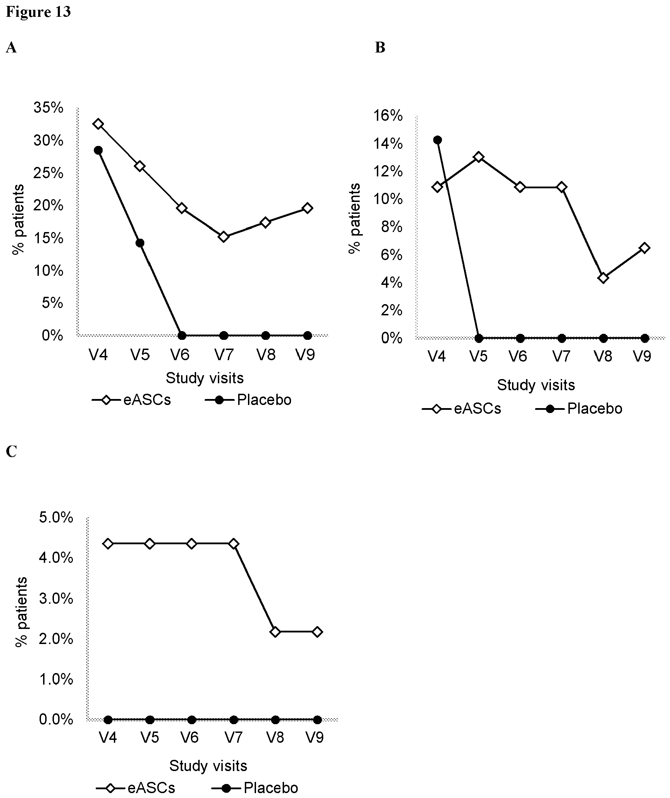

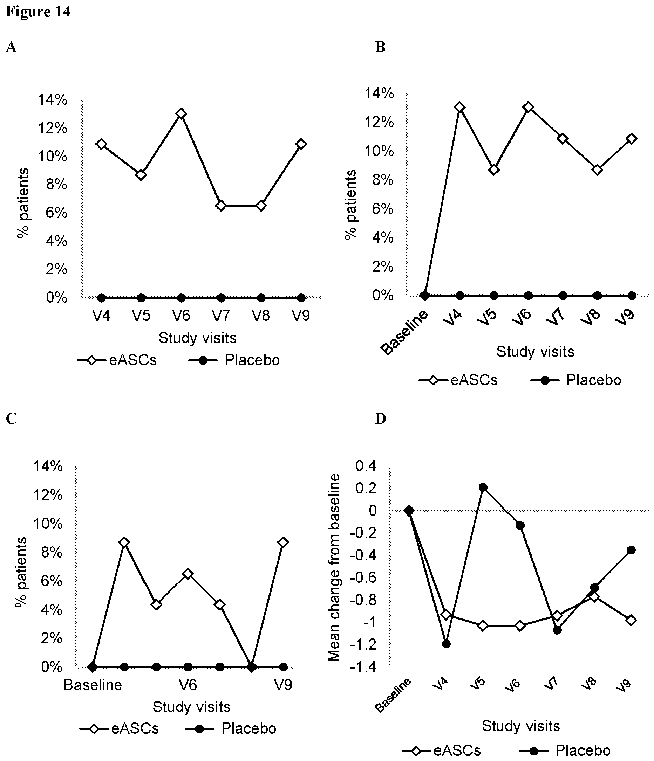

The pooled eASCs-treated patients were compared in an exploratory manner with those treated with placebo. In the ITT population, a higher proportion of patients treated with eASCs, compared with those patients treated with placebo, achieved ACR20, ACR50 and ACR70 (Fig. 13A, B and C, respectively). Results in the ITT and PP populations were similar. When efficacy was assessed in the ITT population according to the EULAR criteria, there was also a trend for a better clinical course in those patients treated with eASCs, in comparison with those treated with placebo. Figure 14A shows the proportion of patients who achieved good response according to EULAR response (DAS28-ESR present score ≤3.2 and improvement >1.2), and Fig. 14B and 14C those showing low disease activity (DAS28-ESR<3.2) and those in clinical remission (DAS28-ESR<2.6), respectively. Of note, in contrast to pooled eASC-group, no patients in the placebo group showed good EULAR response, low disease activity or were clinical remission at any time point. DAS28-ESR improvements from baseline were sustained in patients treated with eASC, whereas the placebo arm showed a fluctuating response (Fig. 14D). DAS28-CRP results were similar to those of DAS28-ESR. EULAR responses in the PP population did not differ from those obtained in the ITT population.

Overall, the clinical response rates, evaluated by ACR scores, were higher in patients treated with eASCs than in patients treated with placebo. Evidence of efficacy was also found using the outcomes good EULAR response, low disease activity or clinical remission, in contrast to what happened in the placebo group, where no patients were responders at any time point. Patients treated with eASCs showed a sustained improvement from baseline over time in DAS28-ESR.

Conclusion: Clinical efficacy assessments showed a trend for a better clinical course in the group of patients treated with eASCs, in comparison with those treated with placebo. ASC treatment is particularly effective in patients with moderate rheumatoid arthritis, defined by having DAS28 levels below 4.5 and CDAI levels below 22, even after biologics failure. The subset of patients having low CDAI levels (below 22) showed the best response to ASC treatment, compared to the subset with high CDAI levels (between 22 and 40).

ASC treatment is therefore particularly useful in patients with moderate rheumatoid arthritis, having a DAS28 score of >3.2 to ≤5.1, a CDAI score of >10 to ≤22 and/or a RAPID3 score of >6 to ≤12, in particular those having a CDAI score of > 10 to ≤22.

### Safety

The intravenous infusion of eASCs was in general well tolerated, without an evident dose-related toxicity at the dose range studied. Intensity of AEs was assessed following the Common Terminology Criteria for AEs, version 4.0, of the National Cancer Institute, ranging from 1 to 5 (National Cancer Institute NIoH, U.S. Department of Health and Human Services, Common Terminology Criteria for Adverse Events (CTCAE) Version 4.0. 2010 [cited 2014]; available from http://evs.nci.nih.gov/ftp1/ CTCAE/CTCAE4.03_2010-06-14_QuickReference_5x7.pdf). A total of 141 adverse events (AEs) were reported, of which 41 were considered treatment related. Seventeen patients from the group A (85%), 15 from the group B (75%), 6 from the group C (100%) and 4 from the placebo group (57%) experienced at least one AE. Two treatment-related severe AEs (1 in cohort A and 1 in placebo group), and one treatment-related serious AE (SAE) were reported (cohort A). The most frequent AEs (≥5%) were fever (9; 17%), respiratory infections (8; 15%), headache (6; 11%), urinary tract infections (6; 11%), nausea (5; 9%), arthralgia (3; 6%), asthenia (3; 6%), malaise (3; 6%) and vomiting (3; 6%), and the most frequent treatment-related AEs were fever (7; 13%), headache (4; 8%) and asthenia (3; 6%). By system, the most frequently reported AEs were infections. With the exception of 1 moderate vulvovaginal candidiasis and 1 mild herpes simplex virus infection, no opportunistic infections were reported. Four infections were deemed as related to the study treatment: 2 moderate respiratory infections in the cohort A, and 1 mild urinary tract infection and 1 mild hordeolum, both in the cohort C. Two out of the 41 treatment-related AEs were grade 3 in intensity (severe), 1 in cohort A (lacunar infarction) and 1 in placebo group (asthenia). Three SAEs were reported, all occurring in eASCs treated patients: 1 lacunar infarction, 1 peroneal nerve palsy, and 1 case of pyrexia. Only the lacunar infarction was suspected to be treatment-related. The lacunar infarction was considered a DLT, according to the pre-established definition. This event encompassed 3 consecutive SAEs (2 events of generalised muscle weakness, and 1 event of left hemihypoesthesia and paretic ataxic gait, finally diagnosed as lacunar infarction). These episodes were transient and patient recovered without sequelae. This was the only patient who discontinued the study due to AEs. No abnormal laboratory values were reported and no relevant vital signs abnormalities occurred, other than those related to reported AEs. No malignancies or deaths were reported.

Intravenous treatment with allogeneic eASCs appeared to be, in general, well tolerated. Eighty three per cent of eASC treated patients experienced any adverse event, being most of them unrelated to the study treatment (71%), and of mild or moderate intensity (94%). No life-threatening events (grade 4) or deaths occurred.

Interestingly, there was no apparent relationship between dose and tolerability. In fact, the rate of treatment-related AEs was lower in cohort C than in both cohorts A and B, although this is probably related to the small size of the cohort C. In addition, a potential over reporting of AEs could have occurred in the lower dose cohorts, associated to the unblinded nature of the study concerning safety, due to a potential initial stress associated with the iv injection of stem cells (the study was started administering the lower doses). This suggests that the safety profile of the highest dose level evaluated could be similar to the lower dose levels.

There was one DLT, a lacunar infarction, which occurred in a patient of the cohort A. This SAE encompassed 3 consecutive SAEs (see above), so that muscle weakness, which preceded lacunar infarction, would probably be the real DLT. Nevertheless, as muscle weakness was grade 2, it does not fit in the pre-established definition of DLT. Although there were no other DLTs in patients treated with eASCs, one severe and likely related event was reported in the placebo group (asthenia), which indicates that DLT, as defined in the protocol, may occasionally lead to misclassification of certain AEs as DLT.

Lacunar infarction was the only SAE considered as related to eASCs. It was deemed as likely related because there were no other apparent causes, even though a potential underlying mechanism has not been identified. Therefore, a conservative position was adopted in the causality assessment.

Transient fever was the most frequent treatment-related AE occurred in patients treated with eASCs, as depicted in the literature with MSCs. The mechanisms for fever are not clear but could be related to acute inflammatory reactions to MSCs in sensitive patients.

Patients with RA are at higher risk for serious infections than the general population, and prednisone and biologic agents have been shown to increase this risk. The reported risk of serious infections in patients with RA treated with anti-TNF therapies (RR 95% CI) ranges from 1.05 (0.9-1.2) to 4.6 (1.8-11.9).In this trial, four infections were considered as related to the study treatment, not being any of them was severe or serious.

Thrombotic events have been described in animal models in which very high doses of iv MSCs were administered. However, in this study no venous thrombotic events were reported, and no sign suggesting pulmonary thromboembolism was detected.

In addition, no acute or delayed hypersensitivity reactions or haematological AEs, with the exception of anaemia (3 cases), were reported in patients treated with eASCs.

So far, clinical experience from clinical trials does not indicate that tumorigenicity associated to MSC-based therapies represents a major risk. In this study, no malignancies were found.

Therefore, the results show a lack of short-term serious safety issues associated with the therapeutic administration of mesenchymal stem cells to RA patients.

### Other efficacy outcomes

The SF-36 and the physical and mental subscales improved in both groups, without statistical differences among them. RA MRI score (RAMRIS) improved from screening to 6 months, but comparisons among the treatment groups were not statistically significant either.

### EXAMPLE 8

A clinical trial is conducted in a patient population having rheumatoid arthritis, wherein said patients are diagnosed for less than one year and have previously received treatment with at least methotrexate, optionally said patients may have previously received other DMARD treatments such as cyclosporine, hydroxychloroquine, leflunomide, minocycline, and sulfasalazine as well as NSAIDs. If taking methotrexate, leflunomide, or sulfasalazine, patients must have been treated for at least 16 weeks and on a stable dose (oral methotrexate ≤ 25 mg/week; parenteral methotrexate ≤ 20 mg/week; leflunomide ≤ 20 mg/day; sulfasalazine ≤ 3 g/day) for at least 4 weeks prior to the start of treatment and throughout the study. If taking oral corticosteroids, patients must be on a stable dose of prednisone ≤ 10 mg/day or equivalent for at least 1 month prior to screening. If taking NSAIDs, must be on stable dose for at least 2 weeks prior to screening.

Additionally, in order to be eligible for the study the patients must present EULAR DAS28-ESR activity criteria >3.2 as well as four tender joints to palpation and four swollen joints, based on a 68/66-joint count at screening.

Approximately half of patients enrolled in the study receive treatment with a medicinal product consisting of a suspension of donor-derived (allogeneic) expanded adipose stromal cells (eASCs) in Ringer's lactate solution. The other half receive a placebo consisting of a suspension of Ringer's lactate solution. Patients in the treatment group receive 3 doses, at weekly intervals, of 4 million cells/kg patient body weight of the medicinal product. Patients in the placebo group receive 3 doses, at weekly intervals, of the placebo. In both cases administration is by means of intravenous infusion.

The medicinal product is a cell suspension in sterile buffer solution containing adipose-derived stromal cells (eASCs) of allogeneic origin in disposable vials, obtained through lipoaspiration from healthy individuals and expanded in vitro. The medicinal product is supplied as a sterile, clear, colourless suspension for intralesional administration, provided in 6 mL vials (suspension of 10 million eASCs per mL of Dulbecco modification Eagle's medium [DMEM] with human serum albumin). The medicinal product will be administered after suspension in Ringer's lactate solution at an infusion rate of 4ml/min.

The study placebo is a Ringer's lactate solution for intralesional administration at an infusion rate of 4ml/min. The corresponding placebo volume (Ringer's lactate solution) will be administered to each subject from the placebo groups. Placebo volume will be calculated according to the subject's weight.

### Medicinal product preparation

The allogeneic eASCs medicinal product consists of a cellular suspension of living adult stromal cells extracted from the subdermal adipose tissue of healthy donors. Subdermal adipose tissue is liposuctioned from the healthy donor and transported to a GMP manufacturing facility. The donation, procurement, and testing are carried out according to the requirements of Directive 2004/23/EC and therefore under Directives 2006/17/EC and 2006/83/EC. ASCs are isolated by digesting the adipose tissue with type I collagenase, followed by centrifugation. The cell pellet obtained is resuspended and lysed in erythrocyte lysis solution and centrifuged. The stromal vascular fraction, resulting from the cell pellet, is placed in cell culture containers in culture medium and antibiotics, and incubated at 37 °C and 5 % CO2 and in a humidified atmosphere. At 24-48 h post-plating, the culture medium is removed to eliminate the non-attached cell fraction. ASCs adhered to the plastic culture plates are expanded under in vitro conditions. Every 3-4 days, the culture medium is changed after reaching 90-95 % confluence and the cells are detached with trypsin/EDTA, collected, centrifuged, and expanded without antibiotics to the required duplication. They are then harvested and cryopreserved until use. Before the appointed administration date, sufficient cryopreserved vials are thawed to provide the required dose for administration. ASCs are recovered from their cryopreserved state by plating and culturing (to confirm viability). On the day when the vials are filled and packaged, the cultures were washed with phosphate buffer solution, and trypsin/EDTA. The ASCs are immediately resuspended in the selected excipients (Dulbecco modification Eagle medium and human albumin serum) to formulate the drug product.

The eASCs are characterized in terms of identity (phenotypic profile), purity, potency, morphology, viability, and cell growth kinetics according to the Guideline on Cell- Based Medicinal Products (EMEA/CHMP/410869/2006) and the Reflection Paper on Stem Cells (EMA/CAT/ 571134/2009).

## Claims

1. A composition comprising mesenchymal stromal cells (MSCs) for use in treating rheumatoid arthritis in a human subject, wherein the disease is moderate rheumatoid arthritis having a CDAI score of >10 to ≤22 and wherein the subject is first treated at not more than about 12 months from first diagnosis or onset of disease symptoms.

2. The composition for use of claim 1, wherein the disease is moderate rheumatoid arthritis having a DAS28 score of >3.2 to ≤5.1, and/or a RAPID3 score of >6 to ≤12.

3. The composition for use of any preceding claim, wherein the subject is treated during an acute phase of the disease and/or wherein the subject is first treated at not more than about 6 months from first diagnosis or onset of disease symptoms.

4. The composition for use of any preceding claim, wherein the MSCs are administered to the subject repeatedly, optionally at weekly intervals.

5. The composition for use of any preceding claim, wherein at least three doses of MSCs are administered to the subject, for example at days 1, 3 and 5, or days 1, 8 and 15 from the first MSC dose.

6. The composition for use of any preceding claim, wherein the subject is treatment refractory to methotrexate, optionally wherein the subject is treatment refractory to a further DMARD, for example wherein the further DMARD is cyclosporine.

7. The composition for use of any preceding claim, wherein the mesenchymal stromal cells are adipose tissue-derived stromal cells, expanded mesenchymal stromal cells, or expanded adipose tissue-derived stromal cells, optionally wherein the MSCs are allogeneic.

8. The composition for use of any preceding claim, wherein the composition comprises between about 0.25 x 10⁶ cells/kg to about 5 x 10⁶ cells/kg of subject weight.

9. The composition for use of any preceding claim, wherein at least about 50% of the MSCs express one or more of the markers CD9, CD10, CD13, CD29, CD44, CD49A, CD51, CD54, CD55, CD58, CD59, CD90 and CD105.

10. The composition for use of any preceding claim, wherein at least about 50% of the MSCs do not express the markers Factor VIII, alpha-actin, desmin, S-100, keratin, CD11b, CD11c, CD14, CD45, HLAII, CD31, CD34, CD45, STRO-1 and CD133.

11. The composition for use of any preceding claim, wherein the MSCs are administered in a pharmaceutically acceptable carrier and/or a diluent.

12. The composition for use of any preceding claim, wherein the MSCs are administered systemically.

13. The composition for use of any preceding claim, wherein the MSCs are administered via the intravenous, intralymphatic, subcutaneous, intracutaneous, intramuscular, intraarticular, intrasynovial, intrasternal, intrathecal, intralesional, or intracranial route.

14. The composition for use of any preceding claim, wherein the MSCs are administered in conjunction with one or more further therapeutic agents.

15. The composition for use of any preceding claim, wherein the MSCs are cryopreserved.

## Patentansprüche

1. Zusammensetzung umfassend mesenchymale Stroma-Zellen (MSCs) zur Verwendung in der Behandlung von rheumatoider Arthritis in einem menschlichen Subjekt, wobei die Erkrankung moderate rheumatoide Arthritis mit einem CDAI Wert von >10 bis ≤22 ist und wobei das Subjekt erstmalig nicht später als etwa 12 Monate nach Erstdiagnose oder Beginn der Krankheitssymptome behandelt wird.

2. Zusammensetzung zur Verwendung nach Anspruch 1, wobei die Erkrankung moderate rheumatoide Arthritis mit einem DAS28-Wert von >3,2 bis ≤5,1 und/oder einem RAPID3-Wert von >6 bis ≤12 ist.

3. Zusammensetzung zur Verwendung nach einem der vorhergehenden Ansprüche, wobei das Subjekt während einer akuten Phase der Erkrankung behandelt wird, und/oder wobei das Subjekt erstmalig nicht später als etwa 6 Monate nach Erstdiagnose oder Beginn der Krankheitssymptome behandelt wird.

4. Zusammensetzung zur Verwendung nach einem der vorhergehenden Ansprüche, wobei die MSCs dem Subjekt wiederholt verabreicht werden, optional, in wöchentlichen Abständen.

5. Zusammensetzung zur Verwendung nach einem der vorhergehenden Ansprüche, wobei dem Subjekt mindestens drei Dosen der MSCs verabreicht werden, zum Bespiel an den Tagen 1, 3 und 5, oder an den Tagen 1, 8 und 15 seit der ersten MSC Dosis.

6. Zusammensetzung zur Verwendung nach einem der vorhergehenden Ansprüche, wobei das Subjekt behandlungsrefraktär gegenüber Methotrexat ist, optional, wobei das Subjekt behandlungsrefraktär gegenübereinem weiteren DMARD ist, zum Beispiel wobei das weitere DMARD Cyclosporin ist.

7. Zusammensetzung zur Verwendung nach einem der vorhergehenden Ansprüche, wobei die mesenchymalen Stroma-Zellen Stroma-Zellen aus Fettgewebe, expandierte mesenchymale Stroma-Zellen, oder expandierte Stroma-Zellen aus Fettgewebe sind, optional, wobei die MSCs allogen sind.

8. Zusammensetzung zur Verwendung nach einem der vorhergehenden Ansprüche, wobei die Zusammensetzung zwischen etwa 0,25 x 10⁶ Zellen/kg bis etwa 5 x 10⁶ Zellen/kg Gewicht des Subjekts umfasst.

9. Zusammensetzung zur Verwendung nach einem der vorhergehenden Ansprüche, wobei mindestens etwa 50% der MSCs einen oder mehrere der Marker CD9, CD10, CD13, CD29, CD44, CD49A, CD51, CD54, CD55, CD58, CD59, CD90 und CD105 exprimieren.

10. Zusammensetzung zur Verwendung nach einem der vorhergehenden Ansprüche, wobei mindestens etwa 50% der MSCs nicht die Marker Faktor VIII, Alpha-Aktin, Desmin, S-100, Keratin, CD11b, CD11c, CD14, CD45, HLAII, CD31, CD34, CD45, STRO-1 und CD133 exprimieren.

11. Zusammensetzung zur Verwendung nach einem der vorhergehenden Ansprüche, wobei die MSCs in einem pharmazeutisch akzeptablen Träger und/oder einem Verdünnungsmittel verabreicht werden.

12. Zusammensetzung zur Verwendung nach einem der vorhergehenden Ansprüche, wobei die MSCs systemisch verabreicht werden.

13. Zusammensetzung zur Verwendung nach einem der vorhergehenden Ansprüche, wobei die MSCs auf dem intravenösen, intralymphatischen, subkutanen, intrakutanen, intramuskulären, intraartikulären, intrasynovialen, intrasternalen, intrathekalen, intraläsionalen oder intrakraniellen Weg verabreicht werden.

14. Zusammensetzung zur Verwendung nach einem der vorhergehenden Ansprüche, wobei die MSCs in Verbindung mit einem oder mehreren weiteren therapeutischen Wirkstoffen verabreicht werden.

15. Zusammensetzung zur Verwendung nach einem der vorhergehenden Ansprüche, wobei die MSCs kryokonserviert sind.

## Revendications

1. Composition comprenant des cellules stromales mésenchymateuses (MSC) pour utilisation dans le traitement de l'arthrite rhumatoïde chez un sujet humain, sachant que la maladie est de l'arthrite rhumatoïde modérée ayant un taux de CDAI de > 10 à ≤22 et sachant que le sujet est d'abord traité à pas plus d'environ 12 mois à compter du premier diagnostic ou de l'apparition de symptômes de la maladie.

2. La composition pour utilisation de la revendication 1, sachant que la maladie est l'arthrite rhumatoïde modérée ayant un taux de DAS28 de >3,2 à ≤5,1, et/ou un taux de RAPID3 de >6 à ≤12.

3. La composition pour utilisation d'une quelconque revendication précédente, sachant que le sujet est traité pendant une phase aiguë de la maladie et/ou sachant que le sujet est d'abord traité à pas plus d'environ 6 mois à compter du premier diagnostic ou de l'apparition de symptômes de la maladie.

4. La composition pour utilisation d'une quelconque revendication précédente, sachant que les MSC sont administrées au sujet de manière répétée, facultativement à intervalles hebdomadaires.

5. La composition pour utilisation d'une quelconque revendication précédente, sachant qu'au moins trois doses de MSC sont administrées au sujet, par exemple aux jours 1, 3 et 5, ou aux jours 1, 8 et 15 à compter de la première dose de MSC.

6. La composition pour utilisation d'une quelconque revendication précédente, sachant que le sujet est réfractaire au traitement au méthotrexate, facultativement sachant que le sujet est réfractaire au traitement à un DMARD supplémentaire, par exemple sachant que le DMARD supplémentaire est de la cyclosporine.

7. La composition pour utilisation d'une quelconque revendication précédente, sachant que les cellules stromales mésenchymateuses sont des cellules stromales dérivées de tissu adipeux, des cellules stromales mésenchymateuses expansées, ou des cellules stromales dérivées de tissu adipeux expansées, facultativement sachant que les MSC sont allogéniques.

8. La composition pour utilisation d'une quelconque revendication précédente, sachant que la composition comprend entre environ 0,25 x 10⁶ cellules/kg à environ 5 x 10⁶ cellules/kg de poids du sujet.

9. La composition pour utilisation d'une quelconque revendication précédente, sachant qu'au moins environ 50 % des MSC expriment un ou plusieurs des marqueurs CD9, CD10, CD13, CD29, CD44, CD49A, CD51, CD54, CD55, CD58, CD59, CD90 et CD105.

10. La composition pour utilisation d'une quelconque revendication précédente, sachant qu'au moins environ 50 % des MSC n'expriment pas les marqueurs Facteur VIII, alpha-actine, desmine, S-100, kératine, CD11b, CD11c, CD14, CD45, HLAII, CD31, CD45, STRO-1 et CD133.

11. La composition pour utilisation d'une quelconque revendication précédente, sachant que les MSC sont administrées dans un support pharmaceutiquement acceptable et/ou un diluant.

12. La composition pour utilisation d'une quelconque revendication précédente, sachant que les MSC sont administrées de manière systémique.

13. La composition pour utilisation d'une quelconque revendication précédente, sachant que les MSC sont administrées par voie intraveineuse, intralymphatique, sous-cutanée, intracutanée, intramusculaire, intraarticulaire, intrasynoviale, intrasternale, intrathécale, intralésionale, ou intracraniale.

14. La composition pour utilisation d'une quelconque revendication précédente, sachant que les MSC sont administrées en conjonction avec un ou plusieurs agents thérapeutiques supplémentaires.

15. La composition pour utilisation d'une quelconque revendication précédente, sachant que les MSC sont cryopréservées.
